Europäisches Patentamt

(19) European Patent Office

Office européen des brevets

(11) Publication number: **0 133 123**
**A1**

(12) # EUROPEAN PATENT APPLICATION

(21) Application number: **84401548.7**

(22) Date of filing: **23.07.84**

(51) Int. Cl.⁴: **C 12 N 15/00**
**C 12 P 21/00, C 07 K 15/00**
**A 61 K 39/12**
**//C12R1/19**

(30) Priority: **25.07.83 US 517063**
**11.07.84 US 629852**

(43) Date of publication of application:
**13.02.85 Bulletin 85/7**

(84) Designated Contracting States:
**DE FR GB**

(71) Applicant: **MOLECULAR GENETICS, INC.**
**10320 Bren Road East**
**Minnetonka Minnesota 55434(US)**

(72) Inventor: **Pilacinski, William P.**
**6990 Crest Drive**
**Maple Grove Minnesota(US)**

(72) Inventor: **Glassman, Donald L.**
**1417 East 80th Street**
**Bloomington Minnesota(US)**

(72) Inventor: **Krzyzek, Richard A.**
**5305 Woodlawn Blvd.**
**Minneapolis minnesota(US)**

(72) Inventor: **Sadowski, Peter L.**
**12094 Chesholm Lane**
**Eden Prairie Minnesota(US)**

(74) Representative: **Warcoin, Jacques et al,**
**Cabinet Régimbeau 26, avenue Kléber**
**F-75116 Paris(FR)**

(54) Immunogens of papilloma viruses.

(57) This invention describes a process for the production of proteins related to viral capsid proteins of papillomavirus (PV), and to processes and compositions for making and using novel DNA sequences, plasmids and microorganisms to produce the viral proteins.

The present invention utilizes recombinant DNA techniques to insert DNA sequences coding for a capsid protein of bovine papillomavirus (BPV), or portions thereof, into DNA vectors, such that the vector is capable of replicating and directing expression of the BPV DNA in a bacterial host or other single cell system. The resulting recombinant DNA molecule is used to transform host cells and thereby enables production of BPV-related proteins. The proteins so produced are isolated, purified and formulated for use as immunogens in vaccines which protect against infection by papillomavirus.

EP 0 133 123 A1

Croydon Printing Company Ltd.

0133123

TITLE OF THE INVENTION

IMMUNOGENS OF PAPILLOMA VIRUSES.

## 1. FIELD OF THE INVENTION

This invention relates to a process for the production of proteins related to viral capsid proteins of papillomavirus (PV), and to processes and compositions for making and using novel DNA sequences, plasmids and microorganisms to produce viral capsid proteins.

The present invention utilizes recombinant DNA techniques to insert a DNA sequence coding for a capsid protein of bovine papillomavirus (BPV), or a portion thereof, into a DNA vector, such as viral DNA, plasmid DNA or bacteriophage DNA, such that the vector is capable of replicating and directing expression of the BPV DNA in a bacterial host or other single cell system. The resulting recombinant DNA molecule is used to transform host cells and thereby enables production of BPV-related proteins, or portions thereof, by the host cells. The proteins so produced are isolated, purified and modified for use as immunogens in vaccines which protect against infection by papillomavirus.

## 2. BACKGROUND OF THE INVENTION

### 2.1. RECOMBINANT DNA TECHNOLOGY AND GENE EXPRESSION

Recombinant DNA technology involves insertion of specific DNA sequences into a DNA vehicle (vector) to form a recombinant DNA molecule which is capable of replication in a host cell. Generally, the inserted DNA sequence is foreign to the recipient DNA vehicle, i.e., the inserted DNA sequence and the DNA vector are derived from organisms which do not exchange genetic information in nature, or the inserted DNA sequence may be wholly or partially synthetically made. In recent years several general methods have been developed which enable construction of recombinant DNA molecules. For example, U.S. Pat. No. 4,237,224 to Cohen and Boyer describes production of such recombinant plasmids using restriction enzymes and methods known as ligation. These recombinant plasmids are then introduced and replicated in unicellular organisms by means of transformation. Because of the general applicability of the techniques described therein, U.S. Pat. No. 4,237,224 is hereby incorporated by reference into the present specification.

Another method for introducing recombinant DNA molecules into unicellular organisms is described by Collins and Hohn in U.S. Pat. No. 4,304,863 which is also incorporated herein by reference. This method utilizes a packaging/transduction system with bacteriophage vectors (cosmids).

Regardless of the method used for construction, the recombinant DNA molecule must be compatible with the host cell, i.e., capable of autonomous replication in the host cell. The recombinant DNA molecule should also have a marker function which allows the selection of host cells so transformed by the recombinant DNA molecule. In addition, if all of the proper replication, transcription and translation signals are correctly arranged on the DNA molecule, the foreign gene will be properly expressed in the transformed cells and their progeny.

As is characteristic of all viruses which infect eucaryotic cells, papillomavirus requires a eucaryotic host cell system in which to replicate its genome, express its viral genes and generate its progeny. The signals and control elements for DNA replication, gene expression and virus assembly in eucaryotes differ from those of procaryotes. This is of critical importance when attempts are made to express in procaryotic host cells a gene which is naturally expressed only in eucaryotic cells.

These different genetic signals and processing events control many levels of gene expression; for instance, DNA transcription and messenger RNA translation. Transcription of DNA is dependent upon the presence of a promoter which is a DNA sequence that directs the binding of RNA polymerase and thereby promotes transcription. The DNA sequences of eucaryotic promoters differ from those of procaryotic promoters. Furthermore, eucaryotic promoters and accompanying genetic signals may not be recognized in or may not function in a procaryotic system.

Similarly, translation of messenger RNA (mRNA) in procaryotes depends upon the presence of the proper procaryotic signals which differ from those of eucaryotes. Efficient translation of mRNA in procaryotes requires a ribosome binding site called the Shine-Dalgarno (SD) sequence on the mRNA. This sequence is a short nucleotide sequence of mRNA that is located before the start codon (AUG) which encodes the amino-terminal methionine of the protein. The SD sequences are complementary to the 3'-end of the 16S rRNA (ribosomal RNA) and probably promote binding of mRNA to ribosomes by duplexing with the rRNA to allow correct positioning of the ribosome. For a review on maximizing gene expression, see Roberts and Lauer, 1979, Methods in Enzymology 68: 473.

In addition to these different control elements, many eucaryotic genes contain intervening sequences (introns) which are not present in the mature mRNA or the corresponding double stranded complementary DNA (cDNA) coding for the gene. During mRNA processing in eucaryotes, these introns are spliced out of the mRNA prior to translation (for reviews see Crick, 1979, Science 204: 264; Sharp, 1971, Cell 23: 643).

Expression of eucaryotic genes cloned in procaryotic hosts requires that the coding sequence of the gene of interest be available in a form uninterrupted by intervening sequences. Therefore, when eucaryotic genetic sequences which contain intervening sequences are to be cloned in a procaryotic host, isolation of mRNA as the source of the genetic information offers an advantage over the isolation of the relevant DNA sequence. Accordingly, the isolated mRNA is transcribed into single stranded complementary DNA (cDNA) copies which are then enzymatically processed into double-stranded DNA molecules which can be inserted into cloning vectors.

Such recombinant DNA techniques have been recently used for cloning cDNA copies of RNA viral genomes such as poliovirus; vesicular stomatitis virus; and influenza. These techniques also have application in the development of antiviral vaccines (Heiland and Gething, 1981, Nature (London) 292: 851).

Many other factors complicate the expression of eucaryotic genes in procaryotes even after the proper signals are inserted and appropriately positioned. A clear understanding of the nature of these factors and the mechanisms by which they operate is presently lacking. However, one such factor is the presence of an active proteolytic system in E. coli and other bacteria. This protein-degrading system appears to selectively destroy "abnormal" or foreign proteins such as eucaryotic proteins. A tremendous utility, therefore, would be afforded by the development of a means to protect eucaryotic proteins expressed in bacteria from proteolytic degradation. One strategy is to construct hybrid genes in which the eucaryotic sequence is ligated in phase (_i.e._, in the correct reading frame) with a procaryotic gene resulting in a fusion protein product (a protein that is a hybrid of procaryotic and foreign or eucaryotic amino acid sequences).

Construction of hybrid genes was the approach used in the molecular cloning of genes encoding a number of eucaryotic proteins, such as somatostatin (Itakura _et al._, 1977, Science _198_: 1056), rat pre-proinsulin (Villa-Komaroff _et al._, 1978, Proc. Natl. Acad. Sci., U.S.A. _75_: 3727), growth hormone (Seeburg _et al._, Nature _276_: 795), and ovalbumin-like protein (Mercereau-Puijalon _et al._, 1978, Nature _275_: 505). Additionally, procaryotic promoters have been ligated to such fusion gene sequences in the case of ovalbumin (Fraser _et al._, 1978, Proc. Natl. Acad. Sci., U.S.A. _75_: 5936) and ß-globin (Guarente _et al._, 1980, Cell _20_: 543). The Guarente _et al._ system involves inserting the _lac_ promoter, including the SD sequence, at varying distances in front of the ATG of the fusion gene. Although the molecular cloning and expression of several eucaryotic genes has been accomplished, this has not heretofore been done for any of the papillomavirus genes encoding capsid proteins. Nor is the state of the art such that expression of foreign or eucaryotic genes in procaryotic host cells may be routinely performed.

## 2.2. VACCINES

There are four basic approaches for the control and therapy of viral infections: (1) vaccines that elicit an active immune response; (2) chemotherapeutic agents that inhibit viral replication; (3) agents that induce the synthesis of interferon; and (4) administration of antibodies for passive immunity. All four can be used to prevent infection, but are more effective when applied early in infection. Vaccination, or active immunization is usually ineffective after infection has begun.

Viruses are small and relatively simple in structure. Viruses contain a central core of nucleic acid (either DNA or RNA) surrounded by a protein coat called a capsid. In many viruses, the capsid is surrounded by a loose membranous envelope which is similar to a cellular membrane in composition. Nonenveloped viruses are referred to as naked capsids. The nucleic acid core of viruses contains, through a complex coding scheme, the information needed for infecting cells of host tissues, replicating the virus genome and producing the protein components of the virion particle.

When an animal is infected by a virus, it reacts to the capsid or envelope protein as a foreign protein. The host may develop immunity, which is manifested by the formation of antibodies and lymphocytes which are capable of recognizing the viral proteins. This may result in inactivation of the virus and lysis of infected cells. Thus, conventional vaccines providing active immunity are prepared by producing large amounts of virus in cell tissue culture or in laboratory animals and then rendering the virus harmless without destroying its immunological properties. Traditionally this is accomplished either by inactivating the infectivity of the virus (i.e., "killing" the virus, usually by treatment with various chemical agents such as formaldehyde) for use in inactivated vaccines, or by selecting an avirulent or attenuated (modified) virus for use in live vaccines (attenuated vaccines). Attenuation is obtained by adapting the virus to unusual conditions (e.g., to different animal hosts and cell cultures) and by frequent passages of large viral inocula to select mutants which have lost virulence. A few vaccines still used in veterinary practice consist of fully virulent infectious virus injected in a site where virus multiplication is of little consequence.

Attenuated virus used in live vaccines are generally excellent immunogens because the attenuated virus multiplies in the host thus eliciting long-lasting immunity. Attenuated virus vaccines induce humoral antibodies directed against both virion and nonvirion proteins. However, a number of problems are encountered with the use of live vaccines, such as insufficient attenuation of the virus, genetic instability of the virus, contamination by adventitious viruses in cell cultures used to grow the vaccine virus, and, finally, the vaccine may be unstable (e.g., heat-labile).

While the use of inactivated vaccines (employing "killed" or inactivated virus that does not multiply) avoids the difficulties encountered with the use of live vaccines, killed viruses do not multiply in the immunized animal and usually produce antibodies directed against only virion structural components. There are two major difficulties with inactivated vaccines. Firstly, it is frequently not possible to inactivate all the virus particles. Secondly, there is the difficulty of producing enough virus to provide the necessary quantity of the relevant antigen. Other difficulties encountered with the use of inactivated vaccines are that the immunity achieved is brief and often requires additional immunizations, the antigenic sites may be altered by the inactivating chemical treatment, and thus be less immunogenic or may induce antibodies that are less effective at neutralizing viral infections, and that these vaccines do not induce satisfactory levels of secretory antibody.

Subunit vaccines contain only the necessary and relevant immunogenic material, such as the capsid proteins of nonenveloped icosahedral viruses or the peplomers (glycoprotein spikes) of enveloped viruses. Subunit vaccines can be made by isolating the relevant subunit from highly purified viral fractions, or by synthesizing the relevant polypeptide. A major advantage of subunit vaccines is the exclusion of genetic material of viral origin and of toxic, contaminating or otherwise undesirable substances of the host or donor. However, at present, production of subunit vaccines using these methods is too expensive for widespread commercial veterinary use. Recombinant DNA technology has much to offer in the production of subunit vaccines; the molecular cloning and host cell expression of viral genes which encode the relevant immunogenic portions of the virus or its proteins can produce sufficient quantities of the relevant polypeptide for use as an immunogen in a subunit vaccine.

Vaccines are often administered in conjunction with various adjuvants. The adjuvants aid in attaining a more durable and higher level of immunity using smaller amounts of antigen in fewer doses than if the immunogen were administered alone. The mechanism of adjuvant action is complex and not completely understood. However, it may involve the stimulation of phagocytosis and other activities of the reticuloendothelial system as well as a delayed release and degradation of the antigen. Examples of adjuvants include Freund's adjuvant (complete or incomplete), Adjuvant 65 (containing peanut oil, mannide monooleate and aluminum monostearate), the pluronic polyol L-121 and mineral gels such as aluminum hydroxide, aluminum phosphate, or alum. Freund's adjuvant is no longer used in vaccine formulations for humans or for food animals because it contains nonmetabolizable mineral oil and is a potential carcinogen; however, the mineral gels are widely used in commercial veterinary vaccines.

## 2.3. PAPILLOMAVIRUSES

Virus particles belonging to the Papilloma genus of the family Papovaviridae have been isolated from lesions of man, cattle, rabbit, horse, dog, sheep, elk, deer, mouse and chaffinch. In addition, uncharacterized virus has been detected in papillomatous lesions of coyote, goat, opossum and pig. Papillomavirus is contagious in the animals in which it naturally occurs and natural infections are generally limited to the skin or mucous membranes. The lesions produced are either hyperplastic or benignly neoplastic. Although originally benign, some lesions may become malignant as in the cases of epidermodysplasia verruciformis in humans, papillomas of the upper alimentary tract in cattle and papillomas of the cottontail rabbit.

Papillomaviruses cannot be grown in cell tissue culture nor have they been successfully produced in laboratory animals. Therefore, conventional methods of vaccine production (as discussed in Section 2.2) cannot be used. In one case (bovine papillomavirus) a vaccine is manufactured from fresh wart tissue; however, its efficacy is questionable.

All papillomaviruses show a marked similarity in physical-chemical properties. All have naked, icosahedral capsids ranging from 50 to 55 nm in diameter and a double-stranded, covalently closed circular DNA molecule from $4.5 \times 10^6$ to $5.5 \times 10^6$ daltons in molecular weight. Immunological cross-reactivity has been demonstrated between human papillomavirus type 1 (HPV-1) and cottontail rabbit papillomavirus (CRPV), bovine papillomavirus types 1 and 2 (BPV-1 and 2), canine oral papillomavirus as well as human papillomavirus type 2 (HPV-2) (Jensen et al., 1980, J. Natl. Cancer Inst. 64: 495-500; Orth et al., 1978, Virology 91: 243-255). DNA-DNA hybridization studies have detected regions of significant sequence homology between the genomes of BPV-1 and 2, HPV-1, and CRPV (Law et al., 1979, J. Virol. 32: 199-207).

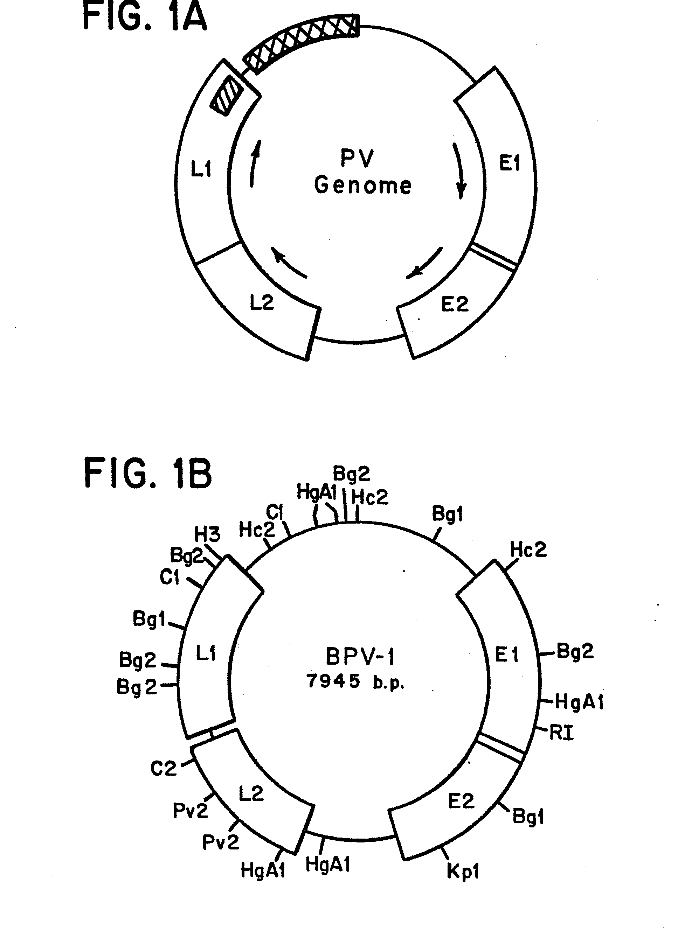

Recently, the complete genomes of BPV-1 and HPV-1 have been sequenced (Chen et al., 1982, Nature 299: 529-534; Danos et al., 1982, EMBOJ 1: 231-236). Analyses of these sequences have shown the great similarity in structure between these two viruses and have allowed construction of a common papillomavirus genome map (see FIG. 1).

## 2.3.1. BOVINE PAPILLOMAVIRUS

Bovine papillomavirus (BPV) produces cutaneous fibropapillomas (warts) on cattle, its natural host. The warts occur either over the head and neck or on the udder and teats. The disease is highly contagious, so, when a herd becomes infected, subsequent generations of cattle are usually involved. The teat warts present problems when using automatic milking machines. Warts in cattle also prevent the animals from being shown, which is a serious problem considering that over 500,000 cattle are raised for show each year. Warts may sometimes become large and invade the animals throat necessitating slaughter.

BPV has also been shown to produce tumors in horses and hamsters (Lancaster et al., 1977, Proc. atl. Acad. Sci. U.S.A. 74: 524-528; Lancaster et al., 1979, Inter-virology 11: 227-233; Olson and Cook, 1951, Proc. Soc. Exp. Biol. Med. 77: 281-284). In addition BPV as well as BPV DNA can transform a number of cell types in culture (Black, 1963, Nature 199: 1016-1018; Boiron et al., 1965, Virology 52: 150-153; Thomas et al., 1964, Nature 202: 709-710). However, complete BPV particles have only been found in wart tissue from the natural host, cattle.

Many attempts have been made to produce BPV particles in cell tissue culture and laboratory animals without success; thus, current BPV vaccines are manufactured from fresh wart tissues obtained from slaughterhouses. The tissue is minced and ground, and the wart material containing proteins and virus particles, is sterilized, diluted to a standard concentration and marketed. The quantity of virus in the vaccine is not determined nor is it a consideration in manufacture. The final product requires 10-50 mℓ of product per immunizing dose and all manufacturers suggest two doses be given to establish immunity.

BPV particles contain only DNA and protein. As in all papillomaviruses this DNA is a double-stranded, covalently closed and supercoiled circular molecule; it is surrounded by protein, forming a capsid with icosohedral symmetry. The capsid is composed of a single protein

subunit with molecular weight of 53,500 daltons. This major capsid protein, designated VPI, constitutes about 75% of the total virus protein. Four host-derived proteins of 15,000, 14,000, 13,000 and 11,280 daltons in molecular weight, along with four minor protein components of 74,000, 70,500, 48,000 and 46,000 daltons in molecular weight are also part of the BPV particle (Meinke and Meinke, 1981, J. Gen. Virol. 52: 15-24).

At least five distinct types of BPV have been described which may differ in one or more of the following criteria: DNA size, DNA sequence, and sites and types of abnormal tissue growths produced (Campo, et al., 1980, Nature 286: 180-182; Lancaster, 1979, J. Virol., 3d, 684-687; Lancaster and Olson, 1978, Virol. 89: 372-379; Pfister et al., 1979, Virology 96: 1-8). Bovine papillomavirus type 1 and type 2 (BPV-1 and BPV-2) are by far the predominant types found in warts on dairy and beef cattle within the United States, and both types are found with about equal frequency. BPV-1 and BPV-2 differ in DNA sequence but these viruses demonstrate immunological cross-reactivity (Lancaster et al., 1979, J. Virol. 32: 684-687; Lancaster and Olson, 1978, 89: 372-379).

### 3. SUMMARY OF THE INVENTION

Methods and compositions are provided for the cloning and expression of papillomavirus (PV) capsid protein genes or portions thereof in microorganisms. Also described are methods for culturing these novel microorganisms to produce PV capsid protein or portions thereof.

In one embodiment of the present invention, the BPV-1 capsid genes were identified in the BPV-1 genome using nucleotide sequence data and restriction maps. Utilizing this information, the BPV-1 capsid protein genes, or portions thereof, were selectively isolated from DNA carrying the entire BPV genome. Restriction endonuclease cleavage was performed at specific sites within the BPV-1 genome so as to isolate the desired fragment containing part or all of the BPV capsid protein genes.

The isolated BPV capsid protein gene or gene fragment was subsequently inserted into a plasmid vector which served as a biologically functional replicon. This plasmid, containing the BPV capsid protein gene, was constructed so as to facilitate both the replication and expression of the BPV capsid protein gene upon transformation of compatible host cells. Additionally, the plasmid provided for a one-step identification of transformed microorganisms actively expressing the BPV capsid protein.

The proteins thus produced may be used in vaccine formulations. Vaccines produced by recombinant DNA technology can address many of the problems involved in the production of conventional vaccines. First, very little virus is needed in order to isolate the relevant genetic sequences which are used in the construction of recombinant DNA molecules. The use of such recombinant molecules to transform appropriate host cells allows for the production of effective vaccines against viruses that cannot be grown in cell tissue culture or in laboratory animals. Second, recombinant DNA vaccines are a type of subunit vaccine, they contain no viral genetic material or toxic, contaminating host substances. Third, since large scale bacterial fermentation can be used, the vaccines can be produced economically in large quantities.

The present invention may be more fully understood by reference to the following detailed description of the invention, examples of specific embodiments of the invention, and the appended figures.

## 4. BRIEF DESCRIPTION OF THE FIGURES

The text which follows may be more easily understood with reference to the following figures, which are not drawn to scale. In all figures, restriction endonuclease recognition sites are indicated by the following abbreviations: BamHI (Bml); BglI (Bg1) BglII (Bg2); BstEII (Bse2); ClaI (Cl); EcoRI (Rl); HgiAI (HgAI); HincII (Hc2); HindIII (H3); HpaI (Hpl); KpnI (Kpl); MstI (Msl); Pst 1 (Psl); PvuII (Pv2); RsaI (Rsl); SmaI (Sml); StuI (Stl); and XmaIII (Xm3).

Figure 1A is a diagrammatic representation of a generalized papillomavirus genome map. El and E2 identify open reading frames (an open reading frame is defined as a sequence of DNA that encodes an RNA sequence which is not interrupted by stop codons) that code for "early", non-capsid viral proteins. Ll and L2 identify open reading frames that code for viral capsid proteins. The hatching identifies a highly conserved coding region. The cross hatching identifies a region with no significant open reading frames. Arrows indicate direction of code expression.

Figure 1B is a diagramatic representation of a genomic map of BPV-1.

Figure 2 is a diagrammatic representation of the steps involved in the construction of pWPl-1. Restriction endonuclease sites are indicated as well as areas of "open reading frame", all or parts of which, may be BPV-1 genes that code for BPV-1 specific proteins.

Figure 3 represents the nucleotide sequences of the L1 and L2 genes. Only the relevant restriction endonuclease sites are indicated. Vertical arrows indicate the ligation sites of the L1 and L2 amino-coding termini to the expression vector pHK413 thus forming recombinant plasmids pC2V-1 and pFU41.

Figure 4 is a diagrammatic representation of the steps involved in the construction of pBPD18 from pWP1-1 and pMG105.

Figure 5 is a diagrammatic representation of the steps involved in the construction of pC2B9 from pBPD18 and pHK413, and the construction of pC2V1.

Figure 6 is a diagrammatic representation of the steps involved in the construction of pBPD18-25 from pBPD18 and pBR322.

Figure 7 is a diagrammatic representation of the steps involved in the construction of pBPT13 from pDR540 and pBPD18-25.

Figure 8 is a diagrammatic representation of the steps involved in the construction of pBPT210 from pBPT13 and pBPD18-25.

Figure 9 is a diagrammatic representation of the steps involved in the construction of pBPT307 from pBPT210 and pBPD18-25.

Figure 10 is a diagrammatic representation of the steps involved in the construction of pCW90 from pWP1-1 and pUC9.

Figure 11 is a diagrammatic representation of the steps involved in the construction of pA2-20 from pCW90 and pHK413.

Figure 12 is a diagrammatic representation of the steps involved in the construction of pFU41 from pA2-20 and pHK413.

## 5. DESCRIPTION OF THE INVENTION

### 5.1. ISOLATION AND CLONING OF THE PAPILLOMAVIRUS GENOME

Papillomavirus particles can be purified from wart tissue using standard isolation techniques, e.g., Meinke and Meinke, 1981, J. Virol. 52:15-24. The virus genomic DNA can be then purified from the viral proteins by standard DNA purification techniques.

In order to provide a continuous supply of large amounts of papillomavirus genomic length DNA, the purified viral DNA can be inserted into a bacterial plasmid which can then be used to transform host cells in order to generate multiple copies. This can be accomplished by cleaving the circular DNA genome at a convenient, unique restriction endonuclease site as determined by restriction endonuclease mapping. This linear DNA molecule can then be inserted into an appropriate cloning vector. Any appropriate cloning vector containing a marker function may be used, for example such vectors include but are not limited to, SV40 DNA, bacteriophage vectors such as lambda gt-WES-lambda B, lambda Charon 28, lambda Charon 4A, lambda gt-lambda BC, lambda GT1-lambda B, or M13mp7, M13mp8, M13mp9 or other plasmid DNA vectors, such as pBR322, pAC105, pVH51, pACYC177, pKH47, pACYC184, pUB110, pMB9, pBR325, ColE1, pSC101, pBR313, pML21, RSF2124, pCR1, or RP4. If the cloning vector does not have restriction sites complementary to the ends of the gene fragment, then the ends of the gene fragment or the vector may be modified.

Once ligated, the recombinant plasmid is used to transform the appropriate host cells. The host cell types used for transformation depend on the cloning vector, that is, the vector must be capable of replication in the host cell and must be otherwise compatible with the host cell. For the purposes of the examples contained herein, E. coli was chosen as the host. Transformants are selected based upon the expression of appropriate gene markers normally present in the vector, such as ampicillin resistance or tetracycline resistance in pBR322. Expression of such marker proteins indicates that the plasmid is intact and is replicating. In the present examples, these recombinant plasmids contain the ampicillin or tetracycline resistance genes, so only clones that grow in the presence of either ampicillin or tetracycline are selected.

### 5.2. IDENTIFICATION OF THE CAPSID PROTEIN GENES

Identification of areas on the genome of a particular papillomavirus that are common to all papillomaviruses allows one to locate the regions on the genome where the capsid proteins are encoded. The generalized papillomavirus genome map (FIG. 1A) shows areas of "open reading frame" labeled E1, E2, L1 and L2,

all or parts of which may be papillomavirus genes that code for papillomavirus specific proteins.

For bovine papillomavirus type 1 (BPV-1) it had been found that within non-natural hosts, where no viral particles are produced, only regions E1 and E2 appear to be processed, while within wart tissues, where many viral particles are produced, regions L1 and L2 are also processed (Antwann and Sauer, 1982, J. Virol. 43: 59-60). Therefore, it is believed that region L1 and/or region L2, or portions thereof, code for the BPV-1 capsid proteins. The results of our experiments, which form the basis for the production of a bovine papillomavirus vaccine, are the first good evidence that regions L1 and L2 do, indeed, code for all, or part of, a BPV-1 capsid protein. The fusion proteins produced herein, which contain portions of regions L1 and L2, induce the production of antibody in experimental animals. The induced antibodies react specifically with, and neutralize the infectivity of whole BPV-1. Therefore, the analogous L1 and/or L2 regions on any papillomavirus should also code for the viral capsid protein.

A number of techniques are available to determine where these common areas are located. Ultimately, the whole papillomavirus genome could be sequenced. The position and direction of all open reading frames, and thus L1 and L2, could then be determined by analysis of the sequence by computer.

Alternatively, areas of significant DNA homology could be used to locate the complementary sequences within the genomes of another papillomavirus. These areas of significant DNA homology are generally located within the open reading frames (e.g., L1, L2, E1 and E2). Therefore,

a fragment of DNA containing the Ll region from a well characterized papillomavirus (e.g., BPV-1) could be used to probe for the Ll region of an uncharacterized papillomavirus genome. The other open reading frames could then be located by their relative positions based upon the generalized papillomavirus genome map.

### 5.3. ISOLATION OF THE PAPILLOMAVIRUS CAPSID PROTEIN GENES

Once the Ll and L2 regions have been located, it is desirable to identify the exact positions of the beginning (initiation codon) and the end (termination codon) of the open reading frames and to identify restriction endonuclease sites convenient to these positions to allow isolation of as much of the open reading frame sequence as possible; this may be accomplished by DNA sequencing and by mapping the restriction endonuclease sites. If convenient sites are not located at a desired position, a large DNA fragment containing the open reading frame may be isolated and excess DNA can be removed using standard techniques, e.g., BAL31 nuclease digestion or exonuclease III followed by S1 nuclease digestion. The DNA ends can be further modified by filling in with E. coli DNA polymerase I or T4 DNA polymerase, and linker ligation (ligating poly dC/dG or poly dA/dT tails or specific restriction linkers onto DNA ends). Generally, the restriction enzymes used for gene isolation are selected with the gene location and cloning vector in mind.

Once convenient restriction sites have been chosen, a large quantity (microgram amounts) of the fragment containing the Ll or L2 gene is isolated. This is accomplished by growing on a large scale, the bacteria containing the viral genome in recombinant plasmids. The plasmid DNA is then isolated and cleaved with the appropriate restriction endonucleases. The desired DNA fragments containing the L1 or L2 sequences are separated according to size and purified by standard techniques including agarose and polyacrylamide gel electrophoresis and column chromatography.

Alternatives to isolating the L1 or L2 gene include but are not limited to chemically synthesizing the gene sqeuence itself (provided the sequence is known) or making cDNA to the mRNA which encodes the L1 or L2 gene. To accomplish this, mRNA specific for L1 or L2 is isolated from homogenized wart tissues. Then the isolated mRNA is converted to a cDNA copy made double stranded and inserted directly into a cloning vector (see Maniatis et al., 1982, Molecular Cloning, A Laboratory Manual, Cold Spring Harbor Laboratory, Ch. 6 and 7).

### 5.4. INSERTION OF THE PAPILLOMAVIRUS CAPSID PROTEIN GENES INTO EXPRESSION VECTORS

Once isolated, the papilloma gene or gene fragment (e.g., L1 or L2 or a portion thereof) is inserted into an appropriate expression vector, i.e., a vector which contains the necessary elements for transcription and translation of the inserted gene.

In order to obtain efficient expression of a foreign gene, an expression vector must have the specific initiation signals required for efficient gene transcription and translation in procaryotic cells. RNA polymerase normally binds to the promoter and initiates transcription of a group of linked genes and regulatory elements, called an operon. Promoters vary in their "strength", i.e., their ability to promote transcription. For the purpose of molecular cloning it is desirable to insert strong promoters in order to obtain a high level of transcription and, hence, expression of the gene. In the present invention, the lac promoter or a hybrid trp-lacuv5 (tac) promoter was used. However, depending upon the host cell system utilized, any one of a number of other suitable promoters may be inserted. For instance, when cloning in an E. coli host cell system, any of the promoters isolated from E. coli, its bacteriophages or plasmids may be used. Additionally, E. coli promoters produced by recombinant DNA or other syntnetic DNA techniques may be inserted to provide for transcription of the inserted gene. More specifically, the $P_R$ and $P_L$ promoters of bacteriophage lambda direct increasingly higher levels of transcription of adjacent DNA fragments. In addition, the recA promoters from E. coli provide high levels of gene transcription of adjacent fragments.

Protein translation is initiated as the result of interaction between two mRNA sites: the ribosome binding site (Shine-Dalgarno [SD] sequence on the DNA complement) and the initiation codon AUG (ATG, on DNA). In the specific examples of the invention the translation initiation signal, specifically the SD-ATG combination, is derived from the E. coli lac gene or bacteriophage lambda. Other suitable SD-ATG combinations that can be uitilized by E . coli ribosomes may be employed, such as the SD-ATG combinations associated with the cro or N genes of bacteriophage lambda, or from the E. coli tryptophan E, D, C, B, or A genes. Additionally, any SD--ATG combination produced by recombinant DNA or other synthetic techniques may be used.

In addition to transcription-translation signals, an expression vector must also have unique restriction endonuclease sites in correct position to allow insertion of the desired DNA fragment. If the desired site is not present, the plasmid may be modified by adding synthetic DNA "linkers" that contain the correct complementary or blunt ends.

The ligated recombinant plasmids containing the L1 or L2 gene are used to transform single-cell hosts such as bacteria. Transformants are screened for papillomavirus protein production by immunoassay. In the preferred embodiment a polypeptide of the predicted molecular weight (based upon the length of the cloned DNA sequence) is identified (e.g., by gel electrophoresis of the proteins produced by the transformants) that reacts with antibody directed against papillomavirus.

In an example of the present invention, a bacterial transformant containing the L1 gene in a recombinant plasmid was identified by the production of a protein of expected size by clones of the transformant. This protein reacted with antibody generated against whole BPV-1 as measured by immune precipitation.

5.5. EXPRESSION OF PAPILLOMAVIRUS CAPSID PROTEIN GENES

Two or more different genes may be joined together using standard DNA cleaving, end modifications and joining techniques (e.g., restriction endonuclease, BAL31 nuclease, ExonucleaseIII, S1 nuclease, E. coli DNA polymerase I digestions, G-C or A-T tailing using terminal deoxynucleotidyl transferase, or ligation with synthetic DNA "linkers"). If gene sequences are constructed such that their coding sequences are ligated in phase with no interrupting termination signals, then subsequent host cell translation of the ligated genes results in production of a fusion protein containing parts of each gene.

Gene ligation may allow increased expression of the desired gene product. Highly efficient translation may require the presence or absence of specific regions of sequence complementarity "downstream" from the ATG. Genes which are expressed at high levels (e.g., E. coli ß-galactosidase, recA, trp and bacteriophage lambda cro) may have the desirable sequences downstream from its ATG. Gene ligations incorporating the amino-terminal portion of genes which are expressed at high levels may allow more efficient translation of the desired gene ligated to it.

Gene ligation may also increase the stability of the desired product, i.e. the fusion protein. In bacteria, foreign proteins, especially those of eucaryotes and their viruses, are generally more susceptible to proteolytic degradation than are native procaryotic proteins, therefore, fusion to larger native proteins may protect the desired polypeptide from degradation.

In the example of the present invention, the L1 and L2 regions were ligated to the E. coli ß-galactosidase gene and/or the lambda cro gene resulting in cro/L1/ß-galactosidase, cro/L1 and cro/L2/ß-galactosidase fusion protein production, respectively. However, any other large procaryotic protein (e.g., E. coli, Trp) depending on the particular bacterial host, could be used.

The recombinant plasmid DNA is used to transform bacteria. Transformants may be screened by looking for the production of a new protein of calculated size (extrapolating from the known DNA sequence) or by the immune activity of the papillomavirus portion of the fusion protein (e.g., gel electrophoresis and/or immunoassay).

## 5.6. EVALUATION OF PAPILLOMAVIRUS CAPSID FUSION PROTEINS

Once the desired transformants are identified, analyses of the immunoreactivity and antigenicity of the products expressed by the transformants (e.g., fusion proteins or polypeptides expressed from the L1 or L2 regions) are required. Immunological analyses of these expression products are especially important because the ultimate goal is to use the products so produced in vaccine formulations. The analysis of immunoreactivity is most easily carried out using antisera directed against papillomavirus, whereas immunogenicity and antigenicity may be evaluated by determining test animal antisera titers which develop in response to immunization with the product and the ability of the antisera so produced to neutralize papillomavirus infection.

Ultimately, clinical studies must be performed on the host animal. These studies will determine whether the vaccine products can inhibit wart formation by clinically induced infection with purified papillomavirus.

In the examples of the present invention, the cro/L1/ß-galactosidase protein was found to react with antibody directed against BPV-1; and antibody raised against both cro/L1/ß-galactosidase and cro/L2/ß-galactosidase fusion proteins reacted with whole BPV-1 and neutralized BPV-1 in an in vitro infectivity assay. Additionally, vaccination of cattle with cro/L1/ß-galactosidase fusion proteins inhibited wart formation in animals infected with purified bovine papillomavirus.

## 5.7. PURIFICATION OF PAPILLOMAVIRUS PROTEINS OR FUSION PROTEINS

Transformants containing the desired papillomavirus genetic sequences are cultured and the polypeptides or proteins produced after induction of the promoter are isolated from such cells or from the medium if the proteins are excreted. The proteins can be isolated and purified either by standard chromatography including ion exchange, affinity or sizing resins, by centrifugation, or by any other standard technique for the purification of proteins.

Fusion proteins may be isolated from aggregates in the host cell and allow the use of a particularly easy method for isolating them. Such a method involves the separation, extraction and/or purification of aggregate-forming proteins from the protein mixtures such as lysates of cell cultures by utilizing, consecutively, the steps of cell disruption and differential centrifugation. Highly purified aggregate protein can be obtained at this point. Additionally, the aggregate proteins may be solubilized and reprecipitated. Solubilization of the aggregate-forming proteins may be accomplished with a solvent that is both a strong hydrogen acceptor and a strong hydrogen donor (or a combination of solvents each having one of these properties), and precipitation of the aggregate-forming proteins by dilution with a compatible buffer. Suitable protein solvents include, but are not limited to urea (from about 4 M to about 8 M), formamide (at least about 80%, volume/volume basis), and guanidine hydrochloride (from about 4 M to about 8 M). Although guanidine hydrochloride and other similar agents are denaturants, studies indicate that this denaturation is reversible and that renaturation may occur upon removal (by dialysis, for example) or dilution of the denaturant, allowing reformation of an immunologically and/or biologically active protein.

An example of the fusion protein isolation technique utilized in this invention is outlined as follows:  the cell pellet is quick frozen using dry ice/methanol, weighed, and 3-4 g of cells are resuspended in at least 25 mℓ of a buffer solution (e.g., 50 mM Tris-HCl [tris hydroxymethylaminomethane-hydrochloride], pH 8.0, 2 mM EDTA [ethylenediaminetetraacetic acid] and 200 mM NaCl).  That is, the cells are suspended in a buffer solution at an approximate concentration of from about 100 to 200 grams of cells/liter.  Concentrations less than about 160 grams/liter are preferred.  To this suspension lysozyme is added to a final concentration of about 130 µg/mℓ and the resulting mixture is allowed to stand at 4°C for 20 minutes with occasional snaking. Nonidet-P40 (NP-40, Shell trademark, polyoxyethylene [9] P-tert-octylphenol), a non-ionic detergent used to solubilize membranes, is added to a final concentration of about 0.1% and the solution mixed.  Then, the suspension is ground for approximately 1 minute using a Polytron homogenizer (Brinkman Instruments, Westbury, NY) or equivalent.  Cell disruption may be accomplished by wide variety of mechanical or non-mechanical means or any combination thereof.  Thus, the step of cell disruption includes, but is not limited to the use of a Polytron homogenizer, enzymes and chemicals such as lysozymes and detergent, a Gaulin Laboratory Homogenizer (Gaulin Corporation, Everett, MA), or DYNO-Mill (Impandex, Inc., Maywood, NJ).

The suspension is centrifuged at 8,000 x g for 30 minutes, and the pellet resuspended in wash buffer, e.g., 20 mM Tris-HCl (pH 7.2), 1 mM EDTA, 150 mM NaCl and

0133123

2% Triton-X 100 (polyoxyethylene [9-10] p-tert-octylphenol, non-ionic detergent), and ground using the Polytron homogenizer. This step of centrifugation, washing, and homogenizing may be repeated to further wash the pellet and remove as much cellular debris as desirable. This pellet of aggregates may be resuspended in an appropriate buffer (such as phosphate buffered saline [PBS]: 20 mM sodium phosphate, pH 6.8, 150 mM NaCl) and used directly as an immunogen, or the fusion protein in the pellet may be solubilized as described below.

In order to solubilize the aggregates the suspension is centrifuged, the supernatant removed completely and the pellet resuspended in about one-fifth volume of 6 M guanidine hydrochloride in distilled water or any appropriate buffer). For instance, 3 g of cells washed with 25 ml of buffer should be resuspended at this step in 5 ml of 6 M guanidine hydrochloride solution. It may be difficult to resuspend the pellet at this stage and sonication or homogenization may be required in order to obtain a homogeneous solution. The solution is allowed to stand at 22°C for 20 minutes and is then centrifuged at 8,000 x g for 30 minutes to remove debris, saving the supernatant which at this point contains the solubilized fusion protein.

The fusion protein is precipitated from the guanidine hydrochloride supernatant by the addition of about four volumes of water or aqueous buffer (almost any aqueous buffer may be used at this stage). The addition of a small amount of non-ionic detergent, such as 0.5% NP-40 or Triton X-100 may be helpful in some cases. This suspension is allowed to stand at 4°C for about 30 minutes and is then centrifuged at 2,000 x g for 10 minutes. The supernatant is discarded, the pellet (containing the fusion protein precipitate) is resuspended in an appropriate buffer, e.g., phosphate buffered saline (PBS) in an appropriate volume. Brief sonication or homogenization may aid in obtaining a homogeneous suspension or slurry (since aggregate-forming proteins are insoluble in water).

It may be desirable to remove any remaining DNA from the suspension of aggregates or the fusion protein precipitate before using either as an immunogen. To this end the suspension of aggregates or fusion protein precipitate is pelleted and resuspended in wash buffer containing no detergent. To either suspension a final concentration of about 10 mM $MgCl_2$ and 2 µg/mℓ Deoxyribonuclease I (P.L. Biochemicals, Milwaukee, WI) is added. After incubation at 37°C for 30 minutes, the suspension of aggregates or fusion protein precipitate is pelleted by centrifugation, washed with buffer, repelleted, and resuspended in fresh buffer, thus removing most of the deoxyribonuclease.

## 5.8. FORMULATION OF A PAPILLOMAVIRUS VACCINE

The purpose of this invention is to produce, by recombinant DNA techniques, polypeptides related to papillomavirus structural proteins which may be used as immunogens in a subunit vaccine to protect against papillomavirus infections. If the protein product elicits an immune response that neutralizes papillomavirus in vitro, it would be expected that this same protein would elicit an immune response capable of neutralizing the relevant virus in vivo. Vaccines made from genetically engineered immunogens should be safer than conventional vaccines made from attenuated or killed virus because there is no risk of infection of the recipient.

Although the papillomavirus/ß-galactosidase fusion protein product itself may be useful in a vaccine formulation, it may be advantageous to first remove and/or modify all or part of the ß-galactosidase moiety in order to produce a more effective papillomavirus-related immunogen.

While whole cells or crude extracts of induced transformants may be used to vaccinate animals, the genetically engineered papillomavirus polypeptide or fusion protein may also be isolated and purified from the host cells using standard protein isolation techniques or techniques previously described (see Section 5.7). In fact, any of the methods used to isolate aggregates from host cells may be used in vaccine formulations. The final purified product may then be adjusted to an appropriate concentration and formulated with any suitable vaccine adjuvant and packaged for use. Suitable adjuvants include but are not limited to surface active substances, e.g., hexadecylamine, octadecylamine, lysolecithin, dimethyl-dioctadecylammonium bromide, N,N-dioctadecyl-N´-N´bis(2-hydroxyethyl-propane diamine), methoxyhexadecylglycerol, and pluronic polyols; polyanions, e.g., pyran, dextran sulfate, poly IC, polyacrylic acid, carbopol; peptides, e.g., muramyl dipeptide, dimethylglycine, tuftsin; oil emulsions; and alum. Finally, the protein product may be incorporated into liposomes for use in a vaccine formulation, or may be conjugated to polysaccharides or other polymers.

The genetically engineered DNA molecules described herein allow great flexibility for vaccine production. For example, a vaccine could be formulated using a papillomavirus-related protein produced by transformants containing any portion of the genetic sequences of papillomavirus encoding a structural protein or multiple copies of such sequences in tandem. The papillomavirus genetic sequences (or portion thereof) may be ligated to genes that encode other immunogens so that the fused protein product could be used in the preparation of multivalent vaccines. Additionally, the papillomavirus genetic sequences (or portions thereof) could be ligated to other papillomavirus gene sequences (or portions thereof) in any combination. Finally, the genetic sequences of papillomavirus may be reorganized to increase the immunogenicity of the vaccine. For example, the papillomavirus genetic sequences may be altered so that the protein product presents specific epitopes to the immune system (e.g., an antigenic site of the protein that is normally only partially exposed may be presented to the immune system); or the regions of the genetic sequences that encode immunosuppressive portions of the protein can be deleted; or the regions that merely separate epitopes can be deleted.

## 6. EXAMPLE: BOVINE PAPILLOMAVIRUS

### 6.1. GENERAL PROCEDURES

Unless otherwise indicated in the text, the following subsections describe the general procedures and materials utilized in the practice of this example of the present invention.

### 6.1.1. PLASMID DNA ISOLATION

Host cell bacteria Escherichia coli (E. coli) were transformed (Gautier and Bonewald, 1980, Molec. Gen. Genet. 178: 375) and large (microgram) quantities of plasmid DNA were isolated from cultures of E. coli transformants grown in LB medium (Miller, Experiments in Molecular Genetics, Cold Spring Harbor Press, New York, N.Y., 1972) to which casamino acids have been added (about 2 g per liter) and 0.01 M $MgSO_4$. Plasmids were isolated from cells in late logarithmic stage of growth using the method of Guerry et al. (1973, J. Bacteriol. 116: 1063).

### 6.1.2. CONDITIONS FOR RESTRICTION ENZYME DIGESTIONS

Restriction enzymes used in the present application were obtained commercially. An enzyme unit is defined as the amount required to digest 1.0 µg of lambda DNA in 60 minutes at 37°C in a total reaction mixture of 50 µℓ.

All restriction enzyme total digestions were performed under the following conditions: each 1 µg DNA was incubated with 5.0 units of enzyme at 37°C for 30 minutes in 6 to 20 µℓ buffer. Complete or total digestions were monitored by agarose gel electrophoresis of an aliquot of the reaction mixture. Complete digestion is indicated in each case by the correct size and number of DNA fragments which are detected as discrete bands in the gel.

Partial digestions were accomplished by modifying the conditions used for total digestion as follows: each 1 µg DNA was incubated with 0.1 units of enzyme at 37°C for 15 minute intervals. The fractions selected were those in which an average of one cleavage per DNA molecule was obtained. When restriction enzyme reactions were terminated before electrophoresis, they were terminated by the addition of 0.1% sodium dodecyl sulfate (SDS) or by heating the reaction mixture at 65°C for 10 minutes.

### 6.1.3. RESTRICTION ENZYME BUFFERS

The buffer used for HgiAI digestions consisted of 200 mM NaCl, 10 mM Tris-HCl (pH 8.0) and 10 mM $MgCl_2$.

The buffer used for BstEII, EcoRI, and StuI digestions consisted of: 100 mM NaCl, 50 mM Tris-HCl (pH 7.4), and 10 mM $MgSO_4$.

The buffer used for BamHI, HincII, HindIII, MstI, PstI, PvuII, RsaI, and XmaIII consisted of: 50 mM NaCl, 10 mM Tris-HCl (pH 7.4), 10 mM $MgSO_4$, and 1 mM dithiothrietol (DTT).

The buffer used for BglII, ClaI, and KpnI consisted of: 10mM Tris-HCl (pH 7.4), 10 mM $MgSO_4$, and 1 mM DTT.

The buffer used for HpaI, SmaI and BglI consisted of: 20 mM KCl, 10 mM Tris-HCl (pH 8.0), 10 mM $MgSO_4$, and 1 mM DTT.

It should be noted that whenever two or more restriction endonuclease reactions are performed on DNA, the reaction in low salt buffer is accomplished before the reaction in high salt buffer. If two enzymes require the same buffer, then the reactions may be performed simultaneously.

## 6.1.4.  DNA POLYMERASE REACTION

DNA polymerases catalyze the stepwise elongation of a DNA chain.  Chain growth is in the 5' to 3' direction (i.e., addition of nucleotides occurs at the 3'-terminus) and the sequence of the polymer is determined by that of the template because the incoming nucleotide must be complementary to the template, i.e., form the correct base pair with the template strand.  The known DNA polymerases cannot initiate chain synthesis, thus DNA synthesis requires a primer strand with a free 3'-hydroxyl terminus annealed to a template strand.  Chain elongation proceeds by the nucleophilic attack of the 3'-hydroxyl terminus of the primer on the 5'-phosphate of the incoming nucleotide.  The new chain is base paired and antiparallel to the template strand.  As a result of the DNA polymerase reaction the single-stranded template strand is "filled-in" or converted to a double-stranded DNA molecule.

A second important feature of the DNA polymerases is the "proof-reading" function.  A 3' to 5' exonuclease activity associated with DNA polymerase I acts on nonbase-paired termini and can degrade both single- and double-stranded DNA in the 3' to 5' direction yielding mononucleotides.  Thus an incorrectly added nucleotide is removed before polymerization continues, and the fidelity of the enzyme is further enhanced.  DNA polymerase I also has a 5' to 3' exonuclease activity specific for double-stranded DNA (yielding 5'-mononucleotides and oligonucleotides) which can also excise mismatched regions in DNA.

Proteolytic treatment of DNA polymerase I by the method of Klenow (Klenow et al., 1971, Eur. J. Biochem. 22: 371) yields two fragments, large and small.  The large fragment or Klenow fragment (76,000 daltons) retains the polymerase and 3'- 5' exonuclease activity whereas the small fragment retains the 5'- 3' exonuclease activity. One unit of DNA polymerase I or DNA polymerase I-large fragment (New England Biolabs, Beverly, MA) is defined as the amount converting 10 nmoles of deoxyribonucleotides to an acid insoluble form in 30 minutes at 37°C. The assay conditions for both enzymes are the same except that the reaction mixture for DNA polymerase I contains 67 mM $KPO_4$ (pH 7.5) while the reaction mixture for the Klenow fragment contains 40 mM $KPO_4$ (pH 7.5) in the following buffer: 6.6 mM $MgCl_2$, 1.0 mM β-ME, 2 mM dAT copolymer, 33 μM dATP, 33 μM $^3$H-dTTP and enzyme.

In the present application, when DNA polymerase large (Klenow) fragment was used to fill in single-stranded DNA or cohesive ends that result from restriction enzyme cleavage, the following procedure was employed: restriction enzyme reactions were terminated by heating at 68°C for 10 minutes. To the same reaction mixture a final concentration of 50 μM of each deoxynucleoside triphosphate was added and for each microgram of DNA in the reaction mixture 10-100 units of DNA polymerase Klenow fragment was added. In other words an excess of DNA polymerase Klenow fragment was used to ensure that single-stranded ends were completely filled in.

### 6.1.5. BAL31 NUCLEASE REACTION

BAL31 nuclease is a multifunctional enzyme that contains a highly specific single-stranded endodeoxyribonuclease activity and a processive exonuclease activity that simultaneously degrades both the 3'- and 5'-termini of double-stranded DNA (dsDNA). One unit of nuclease BAL31 (New England Biolabs, Inc.,

Beverly, MA) is defined as the amount required to release 1.0 µg of acid soluble nucleotides from denatured calf thymus DNA (650 µg/mℓ) in one minute at 30°C. The reaction buffer used for BAL31 consisted of: 20 mM Tris-HCl (pH 8.0), 600 mM NaCl, 12 mM CaCl$_2$, 12 mM MgCl$_2$, 1 mM EDTA and DNA. Incubations were at 30°C; BAL31 digests were accomplished by incubating 30 µg of DNA with 0.5 units BAL31 for 1, 2, 4, 6, 8, and 10 minutes. Reactions were terminated by the addition of EDTA to a final concentration of 50 mM or by the addition of an equal volume of phenol equilibrated with 10 mM Tris-HCl (pH 7.5), 5 mM NaCl and 1 mM EDTA (or phenol equilibrated with 20 mM Tris-HCl, pH 7.5, 20 mM NaCl, and 20 mM EDTA).

### 6.1.6. DNA LIGATION

All ligations were accomplished using T4 DNA ligase (New England Biolabs, Inc., Beverly, MA). One unit of T4 DNA ligase is defined as the amount required to yield 50% ligation of HindIII fragments of bacteriophage lambda DNA in 30 minutes at 16°C in 20 µℓ of ligase buffer and a 5'-DNA terminus concentration of 0.12 µM (300 µg/mℓ).

Unless otherwise indicated, DNA ligations were carried out in ligase buffer consisting of: 20 mM Tris-HCl pH 7.8), 10 mM MgCl$_2$ 60 mM ß-ME, 1.0 mM ATP and a DNA concentration ranging from 15-30 µg/mℓ. Ligation reactions were incubated 24 hours at room temperature or 20 hours at 15°C using approximately 300 units of T4 DNA ligase per 10 µℓ reaction volume.

### 6.1.7. GEL PURIFICATION OF DNA FRAGMENTS

After restriction enzyme or nuclease treatment, DNA fragments of varying sizes were separated by gel electrophoresis in low melting temperature agarose (LTA; Bethesda Research Laboratories, Inc., Rockville, MD). After separation the gel was stained with ethidium bromide at 1 μg/ml and the DNA fragments were visualized by exposure to long wavelength ultraviolet light. The desired DNA fragments were extracted from the gel as follows: the band containing the desired DNA fragment was carefully cut out of the ethidium bromide stained gel and melted at 65°C for 20 minutes in the presence of an equal volume of TE (10 mM Tris-HCl, pH 8.0, 1 mM EDTA); the mixture was extracted with an equal volume of TE saturated phenol, followed by an extraction using an equal volume of chloroform-isoamyl alcohol solution at a 24:1 volume ratio; DNA was concentrated by adding 2 volumes of butanol:isopropanol solution at a 7:3 volume ratio, ethanol precipitated and resuspended in TE.

### 6.1.8. ELUTION OF PROTEINS FROM POLYACRYLAMIDE GEL

Cellular proteins were separated in polyacrylamide gels by electrophoresis as described by Laemmli (1970, Nature 227: 680). After separation, about 1 cm was cut away from each side of the gel in a pattern that allowed subsequent realignment of the slice to the gel in its original position. The gel slices were then stained and de-stained as per standard procedure and re-aligned with the remaining portion of the gel. Using the stained gel sides as guides, desired unstained protein was cut out from the large portion of the gel. The gel slice containing the protein of interest was cut into small pieces (2 $mm^2$) and eluted three times, overnight at 37°C in an equal volume of a buffer containing 0.1% SDS and 50 mM $NH_4HCO_3$, pH 8.5.

The eluant was then concentrated by dialyzing under vaccuum using immersible-CX Ultrafilters as the dialysis membrane (Millipore Corp., Bedford, MA). Dialysis was repeated three times, for 12 to 18 hours at room temperature against two liters of a buffer containing 1 mM EDTA, 10% glycerol, 5% methanol, 0.05% Triton X-100 (Rohm & Haas Co., Philadelphia, PA) and 10 mM Tris-HCl, pH 7.2. A final dialysis was done one time, for 6 to 8 hours at room temperature against one liter of a buffer containing 1 mM EDTA, 50% glycerol, 40 mM NaCl and 10 mM Tris-HCl, pH 7.2.

### 6.1.9. BINARY ETHYLENE IMINE INACTIVATION OF BACTERIA

Vaccine materials were inactivated with binary ethylene imine (BEI) as follows: equal volumes of 0.2 M 2-bromoethylamine (Sigma Chemical Co., St. Louis, MO) and 0.4 M NaOH were combined and incubated at 37°C for 60 minutes. Since this reaction generates BEI which is carcinogenic, all manipulations were done in a fume hood while wearing gloves. One-tenth volume of BEI from the first step was added to materials to be inactivated and incubated at 37°C for 4 hours with shaking. Any residual BEI was inactivated by adding 1 M sodium thiosulfate in volume equal to that of BEI in the second step. Inactivated materials were then used for vaccine formulation.

### 6.2. ISOLATION AND CLONING OF THE BOVINE PAPILLOMAVIRUS GENOME

The following subsections describe the molecular cloning of the entire BPV genome using pBR322 as the cloning vehicle. BPV was isolated from wart tissues of infected cattle. The BPV DNA was isolated and cleaved at its unique restriction site, EcoRI. The linearized BPV-DNA (*i.e.*, the entire BPV genome) was then ligated into the EcoRI site of pBR322.

### 6.2.1. PURIFICATION OF BOVINE PAPILLOMAVIRUS DNA

Papillomas were excised and rinsed in 50% glycerol-NaCl at 4°C. The wart tissues were frozen in liquid nitrogen and broken into small pieces by hammering the tissue sandwiched between sheets of aluminum foil. Then 6 g of the papilloma tissue were suspended in 20 mℓ buffer consisting of 50 mM Tris-HCl (pH 7.5), 100 mM NaCl, 10 mM EDTA. The suspension was homogenized in a Polytron grinder (Brinkman Instruments, Westbury, NY) two times for 10 seconds at 4°C at a setting of 6. The homogenate was centrifuged at 1,500 x g for 15 minutes at 4°C, the supernatant was removed and saved (supernatant 1) and the pellet was resupended in 10 mℓ of 0.1 M Tris-HCl (pH 7.5), 0.25% Sarkosyl (N-lauroyl-sarcosine). A 10 mℓ aliquot of Freon (1,1,2-trichloro-1,2,2-trifluoroethane) was added to the suspension which was then homogenized in a Polytron grinder at 4°C for 10 seconds at a setting of 6. The debris and Freon were pelleted by centrifugation at 1,500 x g for 5 minutes at 4°C. The supernatant was combined with supernatant 1 and the mixture was centrifuged at 12,000 x g for 15 minutes at 4°C. The pellet was resuspended in 2 mℓ of 50 mM Tris-HCl (pH 7.5), 100 mM NaCl and used for clinical studies (see Section 6.9.4). The resulting supernatant was centrifuged at 140,000 x g for 1.5 hours at 4°C in order to pellet the virus particles. The pellet containing virus was resuspended in 1 mℓ of 0.1 M Tris-HCl (pH 7.5), 10 mM $CaCl_2$, 10 mM $MgCl_2$ and CsCl (1.33 g/mℓ and centrifuged at 140,000 x g for 40 hours at 20°C in order to band the virus. Fractions (200 μℓ) were collected and the refractive index of each fraction at $O.D._{260}$ and

$O.D._{280}$ was determined in order to identify the fraction which contained the virus. To this end a 5 µℓ aliquot of each fraction was diluted with 150 µℓ of distilled water and the absorbances at $O.D._{260}$ and $O.D._{280}$ were determined; an $O.D._{260}/O.D._{280}$ ratio of 0.7 indicated the fraction which contained the virus. The virus fraction was then dialyzed twice against 0.15 M NaCl, 15 mM sodium citrate, pH 7.0.

DNA was isolated from purified bovine papillomavirus (BPV) by disrupting the virus with 0.5% SDS and extracting the DNA with phenol. The DNA was further purified by ether extraction and ethanol precipitation.

The DNA was determined to be from BPV type 1 (BPV-1) by restriction endonuclease digestion, using EcoRI and HincII, followed by analysis of fragment sizes by agarose gel electrophoresis (restriction enzyme mapping) (Johnson, 1977, Biochem. 16: 4217). A map of the BPV-1 genome is depicted in FIG. 1.

### 6.2.2. MOLECULAR CLONING OF BOVINE PAPILLOMAVIRUS GENOMIC DNA

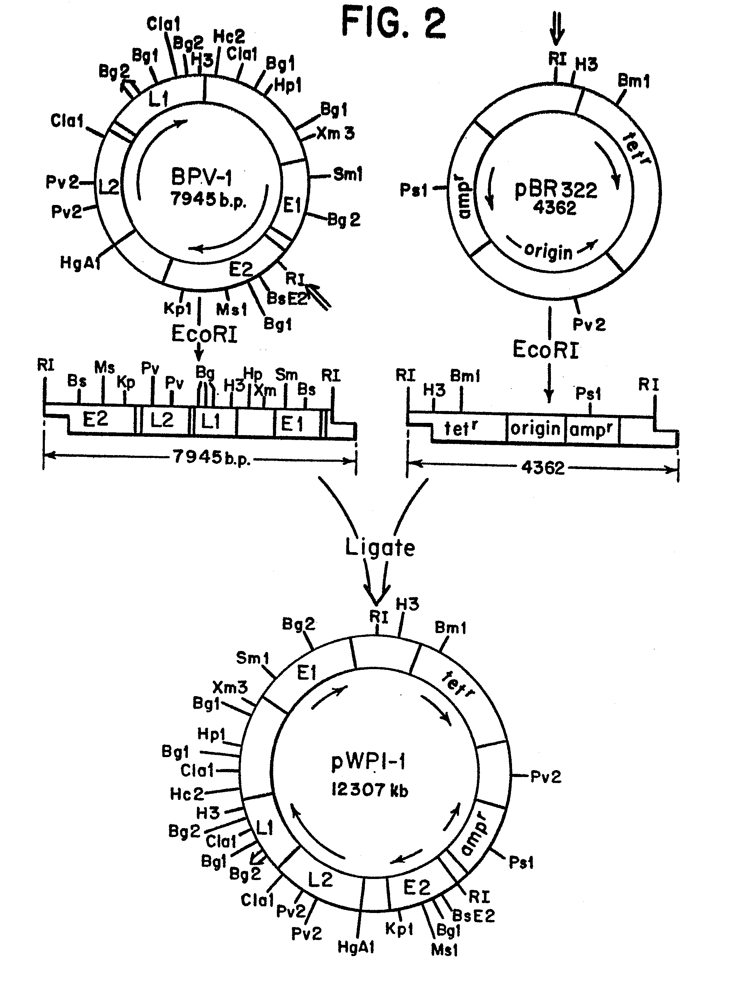

The purified BPV-1 DNA (FIG. 2) was digested to completion with the restriction endonuclease EcoRI. Complete or total digestion of genomic BPV-1 DNA resulted in a single linear DNA fragment with EcoRI cohesive termini. The EcoRI-cleaved BPV-1 DNA was phenol extracted twice, ether extracted twice, ethanol precipitated and resuspended in 10 mM Tris-HCl (pH 8.0), 1 mM EDTA (TE).

Plasmid vector pBR322 (FIG. 2) (Bolivar et al., 1977, Gene 2: 95-113) was isolated and cleaved with EcoRI. The resultant linear pBR322 DNA was phenol extracted and resuspended as described above for BPV-1 DNA.

The cleaved BPV-1 DNA and the cleaved pBR322 plasmid DNA were ligated in a 1:1 molar ratio at a final total concentration of 15 ng/µℓ using T4 DNA ligase as described in Section 6.1.6.

The plasmids resulting from ligation were used to transform E. coli K12, strain MC1000 by the procedure of Kushner (1978 in, Proceedings of the International Symposium of Genetic Engineering) and clones were screened for ampicillin resistance.

Plasmid DNA isolated from ampicillin resistant E. coli clones were analyzed for the presence of BPV-1 DNA by restriction endonuclease mapping using EcoRI. One transformant, designated pWP1-1, contained the entire BPV genomic DNA. Restriction mapping of the recombinant plasmid isolated from clones of this transformant revealed that total digestion with EcoRI yielded two fragments approximately 7945 bp (i.e., the BPV DNA) and 4362 bp (i.e., the pBR322 DNA).

### 6.3. IDENTIFICATION OF THE BPV CAPSID PROTEIN GENES L1 and L2

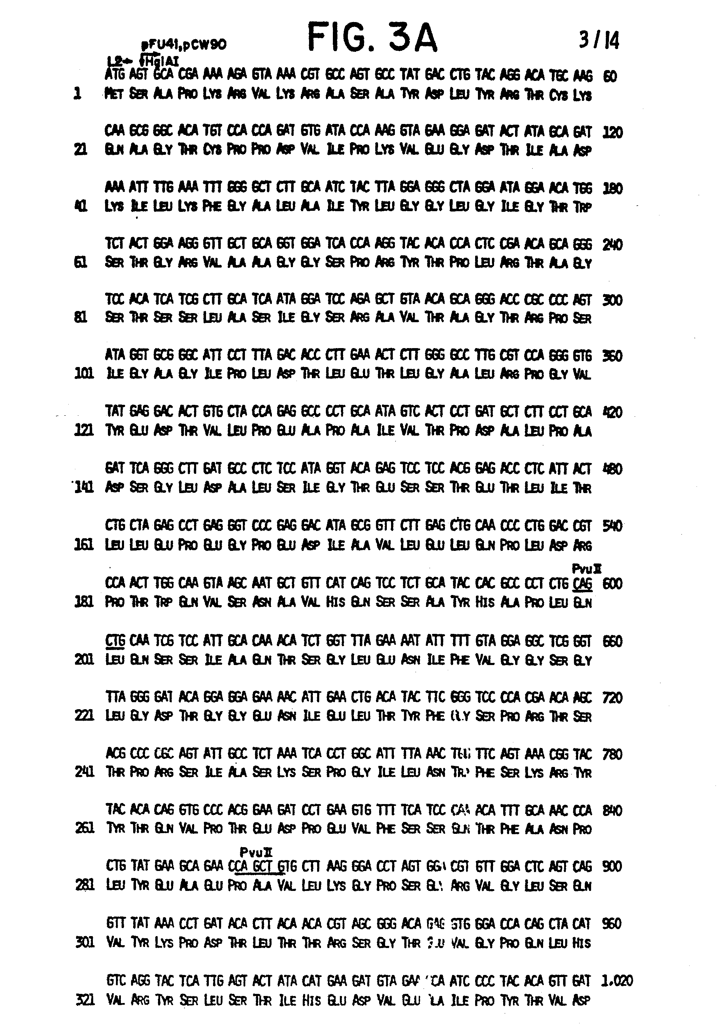

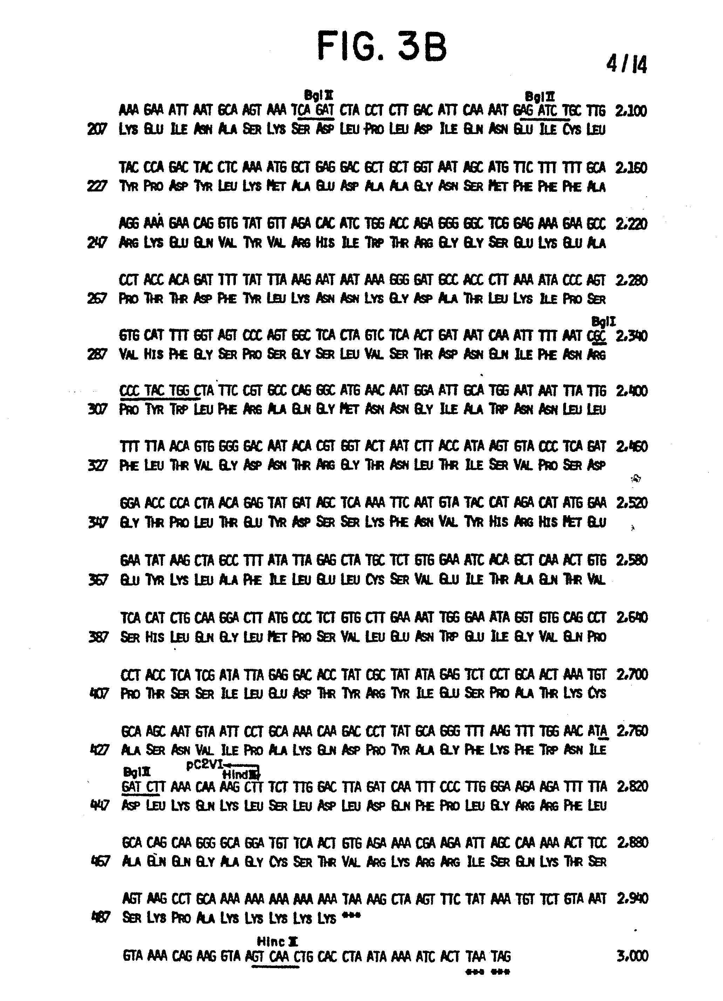

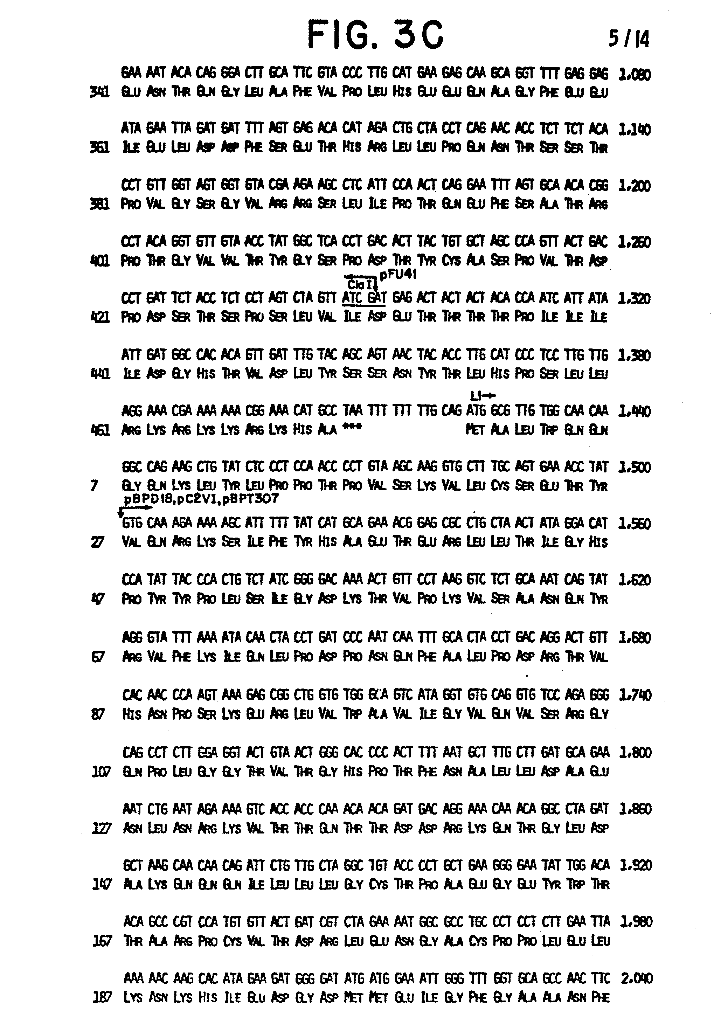

Portions of BPV-1 DNA were sequenced according to the method of Maxam and Gilbert, 1980, Methods in Enzymology 65:499-560). This BPV-1 DNA sequence data as well as that reported in the literature (Chen et al., 1982, Nature 299: 529-534) were analyzed to identify the location of the L1 and L2 regions which were found to be within a 3687 bp fragment defined by restriction endonuclease sites KpnI and HincII. This area of the genome contains two large ATG initiated open reading frames of 1488 bp and 1410 bp corresponding to regions L1 and L2, respectively. The major portions of the L1 and L2 open reading frames are also located within a region identified as not being required for in vitro cell transformation (Lowy et al., 1980, Nature 287:72-74). See also FIG. 3 for nucleotide sequence data of the L1 and L2 genes.

## 6.4. CLONING AND EXPRESSION OF THE L1 GENE

A portion of the L1 gene (hereinafter the L1 gene and portions thereof will be referred to as L1 DNA), lacking the initiation sequence ATG and approximately the first 78 nucleotides of the coding sequence was ligated into a DNA cloning expression vector, pMG105, to form pBPD18. The L1 DNA was inserted so that the protein coding sequence was in the correct reading frame with respect to the initiation ATG of the vector. As a result, subsequent translation of the transcribed mRNA·begins at the initiation sequence ATG of the vector through the L1 gene (lacking its own initiation ATG and the first 68 nucleotides of the L1 coding sequence) to the natural termination signal of the L1 gene.

## 6.4.1. THE EXPRESSION VECTOR pMG105

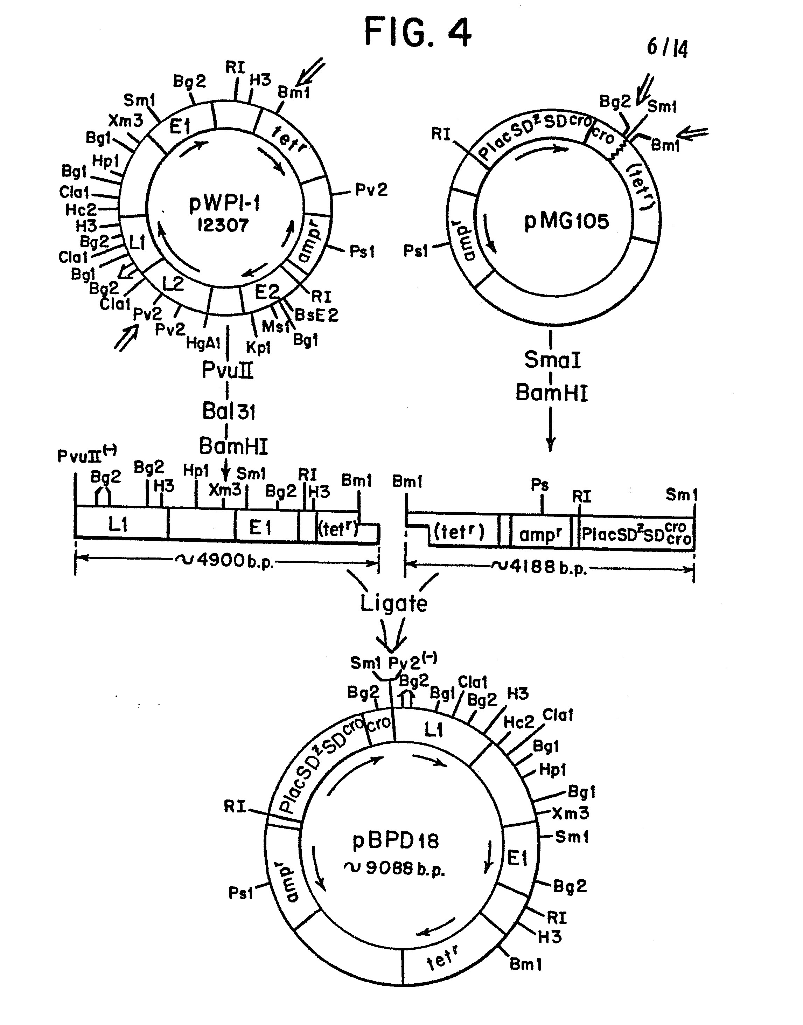

The expression vector pMG105 (see FIG. 4) was constructed from pJS413 (U.S. Patent Applications: Ser. No. 400,028 filed, July 20, 1982; Ser. No. 436,368 filed, October 25, 1982; Ser. No. 449,187 filed, December 13, 1982; and Ser. No. 456,423 filed, January 7, 1982) and pBR325 (Bolivar, 1978, Gene 4: 121-136). It contains the following: the E. coli lac promoter with UV5 mutation, lac and cro ribosome binding sites ($SD^{lacZ}$ and $SD^{cro}$ are represented in all figures as $SD^Z$ and

SD$^{cro}$, respectively) and the carboxy-terminal region of the tetracycline resistance gene (tet$^r$). A BglII, SmaI and BamHI site are located in between the cro and tet$^r$ sequences; these sites allow for insertion of a gene sequence in the correct reading frame with the cro ATG.

pMG105 DNA was digested to completion with restriction endonuclease SmaI followed by BamHI. The Digestion of pMG105 with the enzymes SmaI and BamHI yielded a linear molecule of about 4188 bp which was purified by agarose gel electrophoresis using LTA as described in Section 6.1.7. This DNA molecule had a SmaI blunt end and a BamHI cohesive end.

### 6.4.2. PREPARTION OF THE L1 GENE

Plasmid pWP1-1 DNA was isolated and digested to completion with restriction endonuclease PvuII (see FIG. 4). The PvuII digested DNA was phenol extracted, ether extracted, ethanol precipitated and resuspended in 10 mM Tris-HCl (pH 8.0), 1 mM EDTA (TE).

The PvuII site of interest (i.e., the cleaved PvuII site) within pWP1-1 is located approximately 563 bp upstream from the initiation ATG of the L1 gene; thereore, it was necessary to remove at least the 563 bp before ligating the pWP1-1 fragment to the lambda cro gene of the expression vector. Thus, a minimum of 563 bp was removed using the nuclease BAL31 (Bethesda Research Laboratories, Inc., Rockville, MD) as described in Section 6.1.5. Samples were analyzed by agarose gel electrophoresis to determine the extent of DNA digestion. It was found that incubation for six minutes had removed approximately the correct amount of DNA, and the DNA of this aliquot was ether extracted, ethanol precipitated and resuspended in TE.

The BAL31 digested pWP1-1 DNA was then completely cleaved with the restriction endonuclease BamHI. Completion of the reaction was monitored by agarose gel electrophoresis and fragments, approximately 4900 bp in length, were purified from low melting temperature agarose (LTA; Bethesda Research Laboratories, Inc., Rockville, MD) as described in Section 6.1.7. The purified DNA consisted of a population of DNA fragments of varying sizes because of the random BAL31 digestion.

These purified DNA fragments contained the L1 gene and had a blunt end located at random sites within the ATG-proximal region of the L1 gene and a BamHI cohesive end at the distal end of the fragment. This distal end region also contained the transcription and translation signals and amino-terminal portion of the tetracycline resistance gene (see FIG. 4).

## 6.4.3. LIGATION OF THE L1 GENE AND EXPRESSION VECTOR

The cleaved pMG105 and the 4900 bp fragment containing L1 DNA were then ligated at a 1:1 molar ratio at a final DNA concentration of 24 ng/µℓ. Reaction conditions were as described in Section 6.1.6. The plasmids resulting from ligation were used to transform E. coli K12, strain NF1829, as described previously in Section 6.2.4. The E. coli strain NF1829 is a K-12 MC1000 derivative carrying an F'-lac episome with the lac i$^q$ mutation for lac repressor overproduction. The lac z-gene encoding ß-galactosidase present on the F'-lac episome is inactivated by a Tn 5 (transposon) insertion. Thus, in strain NF1829, the lac promoter must be induced in order

to obtain expression of a gene inserted into the pMG105 plasmid.

### 6.4.4. IDENTIFICATION OF TRANSFORMANTS CONTAINING THE L1 GENE

The PvuII/BamHI DNA fragment (approximately 4900 bp) purified from pWP1-1 in Section 6.4.2. contains the transcription and translation signals and the amino-terminal portion of the tetracycline resistance gene, whereas the BamHI/SmaI pMG105 DNA fragment (approximately 4188 bp) contains the carboxy-terminal portion of the tetracycline resistance gene; therefore, the tet$^r$ gene is restored in the recombinant plasmids resulting from ligation. As a result, transformants were screened for tetracycline resistance.

Recombinant plasmid DNA was purified from tetracycline resistant clones and the isolated DNA was completely digested with the restriction endonuclease BglII. The resulting DNA restriction fragments were then analyzed by agarose gel electrophoresis. The desired constructions contain five BglII DNA fragments; four of these fragments should be the same size in all isolates: 5152 bp, 2515 bp, 670 bp, and 26 bp. The fifth fragment may vary from 641 bp to four base pairs, depending on the amount of DNA removed by the BAL31 nuclease digestion.

Five isolates were identified which had the correct four nonvariable size BglII DNA fragments (5152, 2515, 670 and 26 bp). One isolate, designated pBPD18 (see FIG. 4) had a fifth BglII fragment of approximately 573 bp indicating that about 68 bp (including the ATG of the L1 gene) had been removed from the beginning of the BPV-1 L1 gene.

### 6.4.5. IDENTIFICATION OF TRANSFORMANTS EXPRESSING THE L1 GENE

Transformants containing pBPD18 were identified as expressors of the L1 gene. As explained in Section 6.4.4., the blunt end of the DNA fragment containing L1 gene was produced at random sites within the L1 coding sequence, therefore, ligation to the _cro_ sequence of the expression vector resulted in a mixture of recombinant plasmids. In some of these, the L1 gene was inserted so that the protein coding sequence was in the correct reading frame with respect to the _cro_ initiation ATG of the vector and thus would encode a cro/L1 fusion protein. Since the code is read in triplets of DNA bases, it would be expected that, on the average only one out of three of these isolates would produce a cro/L1 fusion protein. Thus, the five isolates containing BPV-1 DNA from Section 6.4.4. were screened for expression of an antigenically active peptide of BPV by immunoprecipitation techniques using purified IgG directed against BPV-1. Cell lysates of E. _coli_ transformed with plasmid pBPD18 reacted immunologically with the anti-BPV-1 IgG, whereas E. _coli_ transformed with four other isolates or a pMG105 control did not. In addition, SDS-polyacrylamide gel electrophoresis of the immunoprecipitated proteins showed a band corresponding to a protein of the size expected (based upon the length of the cro/L1 DNA sequence). Therefore, pBPD18 was demonstrated to encode a BPV-1-antigenically active cro/L1 protein.

### 6.5. PREPARATION OF pC2V1 WHICH DIRECTS THE PRODUCTION OF CRO/L1/β-GALACTOSIDASE FUSION PROTEIN

As described in the following subsections, a DNA fragment of pBPD18 encoding the transcriptional and translational controls and the cro/L1 fusion protein

without its termination signal (TAA) was ligated to the ß-galactosidase coding sequence (z-gene) of the expression vector pHK413.  After ligation, the translational reading frame was adjusted so that the reading frame of cro/Ll was in phase with that of the z-gene of pHK413.  The resultant plasmid, pC2Vl, directed host cell production of a cro/Ll/ß-galactosidase fusion protein.

### 6.5.1.  THE EXPRESSION VECTOR pHK413

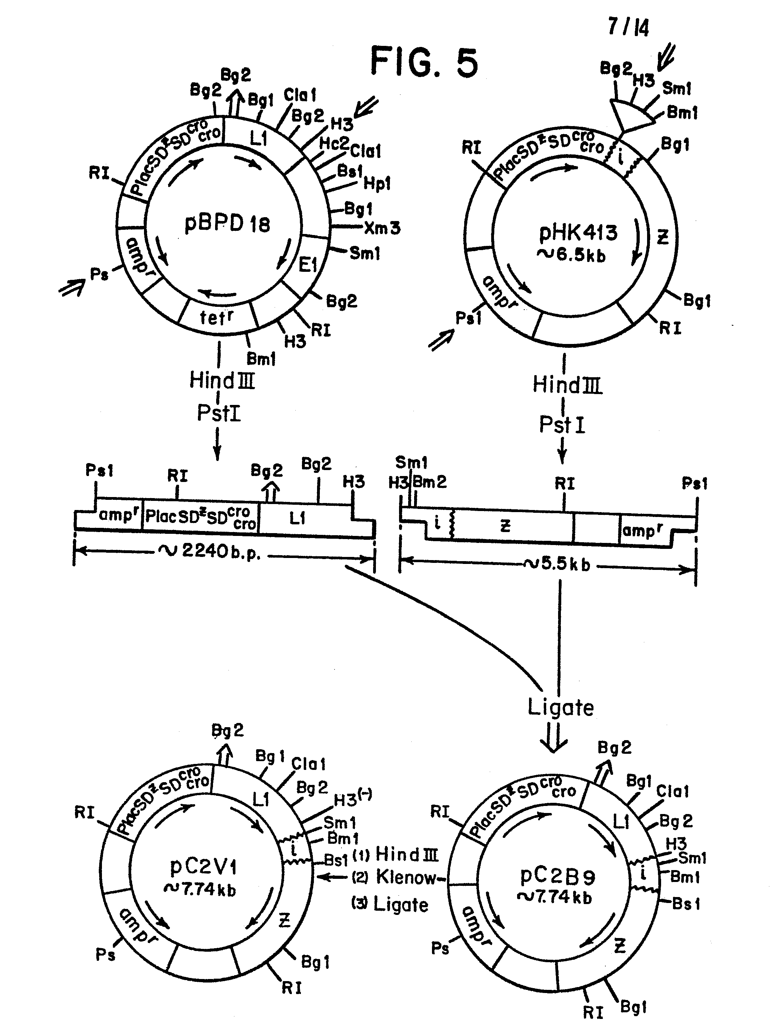

The expression vector pHK413 (6485 bp or about 6.5 k.b.) is a pBR322 derivative which contains the $amp^r$ (ß-lactamase) gene, a lac promoter (Plac), lac and cro ribosome binding sites ($SD^Z$ and $SD^{cro}$, respectively), a chain initiation ATG derived from cro followed by 65 nucleotides of the cro gene, and a modified ß-galactosidase gene (see FIG. 5).  (The pHK413 expression vector is described in detail in a U.S. Patent Application Ser. No. 449,187, filed December 13, 1982, which is herein incorporated by reference).

pHK413 has unique cloning sites which span tne cro-z junction:  BglII, HindIII, SmaI, and BamHI.  The z-gene is not in phase with the cro ATG, thus, the intact plasmid does not direct the production of ß-galactosidase.  However, when a DNA fragment of the appropriate length is inserted into any of these cloning sites on the plasmid, the reading frame of the z-gene may be readjusted with respect to the cro ATG and, provided that the inserted DNA sequence contains no termination signals (e.g., TGA, TAA, or TAG) that are in phase with the cro ATG or z-gene, a ß-galactosidase fusion protein will be produced by host cell transformants.  Accordingly, insertion of a 3n+2 bp DNA fragment between the cro ATG and z-gene of pHK413, results in a readjustment of the reading frames and production of a fusion protein in the host cell transformant, provided the inserted sequences do not contain termination signals in the same reading frame. The inserted DNA sequence and/or the cloning sites of pHK413 may be altered in any fashion in order to readjust the reading frame across the cro-z junction.

Plasmid pHK413 (see FIG. 5) was purified as described in Section 6.1.1. and completely digested with the restriction endonucleases HindIII and PstI. The resulting DNA fragment (about 5.5 k.b.) containing a HindIII cohesive end, the E. coli β-galactosidase gene, the carboxy-terminal portion of the β-lactamase gene (amp$^r$) and a PstI cohesive end was isolated by agarose gel electrophoresis and purified from the LTA gel as described in Section 6.1.7.

### 6.5.2.    INSERTION OF THE L1 GENE INTO pHK413

Plasmid pBPD18 (FIG. 5) was isolated as described in Section 6.1.1. and completely digested with restriction endonuclease HindIII followed by complete digestion with PstI.

After agarose gel electrophoresis, a DNA fragment of about 2240 bp was purified from the LTA as described in Section 6.1.7. This fragment contained a PstI cohesive end, the amino-terminal portion and all transcriptional and translational signals for the amp$^r$ gene, the lac transcriptional signals, the cro translational signals, the cro/L1 sequence and a HindIII cohesive end.

The DNA fragments from pBPD18 and pHK413 were then ligated at a 1:1 molar ratio at a final total DNA concentration of 25 ng/μℓ.  Reaction conditions were as described in Section 6.1.6. The ligated DNA was used to transform E. coli K12, strain NF1829, as described in Section 6.1.1.

### 6.5.3. IDENTIFICATION OF TRANSFORMANTS CONTAINING CRO/L1/ß-GALACTOSIDASE DNA

The PstI/HindIII pBPD18 DNA fragment (Section 6.5.2.) contained the transcription and translational signals as well as the amino-terminal portion of the ß-lactamase gene, which provides ampicillin resistance. The HindIII/PstI pHK413 DNA fragment from Section 6.5.1. contains the portion of the ß-lactamase gene missing from the pBPD18 DNA fragment. When ligated the desired recombinant plasmid generates a complete ß-lactamase gene ($amp^r$); therefore, transformants were selected for ampicillin resistance.

Plasmid DNA from ampicillin resistant isolates was purified as per Section 6.1.1., cleaved by restriction endonucleases HindIII and PstI, and analyzed by agarose gel electrophoresis. One such isolate, designated pC2B9 produced two fragments of about 5500 bp and 2240 bp.

### 6.5.4. ADJUSTING THE TRANSLATIONAL READING FRAME

The DNA sequence of portions of pC2B9 revealed that the ß-galactosidase gene was not in translational reading frame with the cro/L1 DNA sequence. Therefore, it was necessary to adjust the reading frame of the z gene to be in phase with that of the L1 gene. This was done as follows: plasmid pC2B9 was isolated as per Section 6.1.1. and completely digested with restriction endonuclease HindIII. The HindIII cohesive termini were rendered blunt-ended by filling in with the Klenow fragment of E. coli DNA polymerase I as per Section 6.1.4. This resulted in elimination of the HindIII cohesive termini and the creation of blunt ends on the DNA fragment. The blunt ends were joined by ligation as described in Section 6.1.6 (see FIG. 5). The plasmids formed in the ligation reaction were used to transform E. coli K12 strain NF1829 as per Section 6.1.1.

### 6.5.5. IDENTIFICATION OF TRANSFORMANTS EXPRESSING CRO/L1/ß-GALACTOSIDASE FUSION PROTEIN

Transformants from ligation above were screened on MacConkey agar (Difco Labs, Inc., Detroit, MI) containing ampicillin. Colonies producing a ß-galactosidase fusion protein would be a red color and other colonies would be white.

Total cell lysates were prepared from five red colonies. The proteins isolated from these cell lysates were analyzed by SDS polyacrylamide gel electrophoresis (SDS-PAGE). All five isolates produced a large protein of the expected size, about 170,000 daltons molecular weight, while the controls (white colonies) did not. One isolate producing the large fusion protein was designated pC2V1.

pC2V1 plasmid DNA (see FIG. 5) was purified from this isolate as per Section 6.1.1. The pC2V1 plasmid was treated with either HindIII or with a combination of PstI and BamHI as per Sections 6.1.2. and 6.1.3. As expected HindIII did not cleave pC2V1, while digestion with PstI and BamHI produced two fragments of about 5550 bp and 2240 bp.

## 6.6. CONSTRUCTION OF pBPT307 WHICH DIRECTS THE HIGH LEVEL PRODUCTION OF CRO/L1 FUSION PROTEIN

As described in the following subsections, a DNA fragment of pBPD18 encoding the cro/L1 fusion protein was ligated to a DNA fragment of the expression vector pDR540 encoding the transcriptional and translational controls. The resultant plasmid, pBPT307, directed host cell production of a high level of cro/L1 fusion protein.

### 6.6.1. REDUCING THE SIZE OF pBPD18

The construction of pBPT307 required the isolation of a small (332 bp) RsaI generated DNA fragment from pBPD18. pBPD18 contained fourteen RsaI sites, thus, the isolation of the needed fragment would have been difficult. For this reason the size of pBPD18 was reduced by cloning a portion of the plasmid into pBR322.

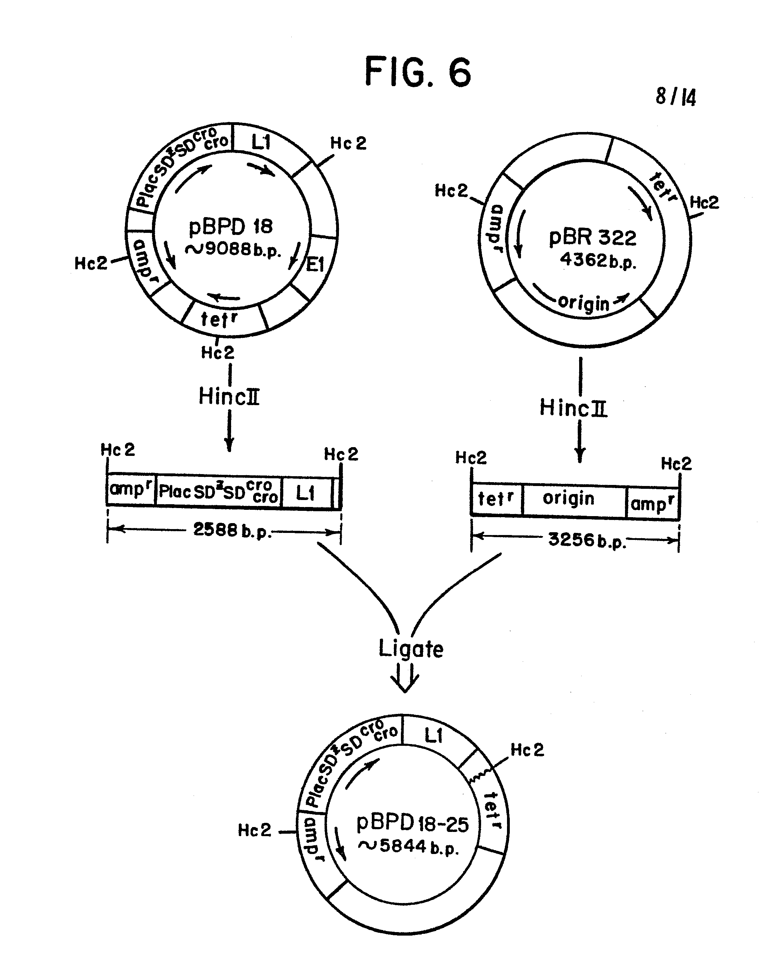

Plasmid pBPD18 (FIG. 6) was isolated as described in Section 6.1.1. and completely digested with restriction endonuclease HincII. After agarose gel electrophoresis, a DNA fragment of about 2588 bp was purified from LTA as described in Section 6.1.7. This fragment contained a HincII blunt end, the amino-terminal portion and all transcriptional and translational signals for the amp$^r$ gene, the lac transcriptional signals, the cro translational signals, the cro/L1 sequence and an HincII blunt end.

Plasmid pBR322 (FIG. 6) was isolated as described in Section 6.1.1. and completely digested with HincII. After agarose gel electrophoresis, a DNA fragment of 3256 bp was purified from LTA as described in Section 6.1.7. This fragment contained an HincII blunt end, the carboxy-terminal portion of the amp$^r$ gene and an HincII blunt end.

The DNA fragments from pBPD18 and pBR322 were then ligated in equal concentration at a final total concentration of 25 ng/µℓ. Reaction conditions were as described in section 6.1.6. The ligated DNA was used to transform E. coli K12, strain MC1000, as described in Section 6.1.1.

### 6.6.2.    IDENTIFICATION OF TRANSFORMANTS CONTAINING CRO/L1 DNA

The HincII pBPD18 DNA fragment (Section 6.1.1.) contained the transcription and translational signals as well as the amino-terminal portion of the ß-lactamase gene, which provides ampicillin resistance. The HincII pBR322 DNA fragment from Section 6.6.1. contained the portion of the ß-lactamase gene missing from the pBPD18 DNA fragment. When ligated, the desired recombinant plasmid generated a complete ß-lactamase gene (amp$^r$); therefore, transformants were selected for ampicillin resistance.

Plasmid DNA from ampicillin resistant isolates was purified as per Section 6.1.1., cleaved by restriction endonuclease HincII, and analyzed by agarose gel electrophoresis. One such isolate, designated pBPD18-25 (FIG. 6) produced two fragments of about 3256 bp and 2588 bp, which indicated the correct construction had been obtained.

### 6.6.3. INSERTION OF THE AMINO-TERMINAL PORTION OF THE CRO/L1 GENE INTO pDR540

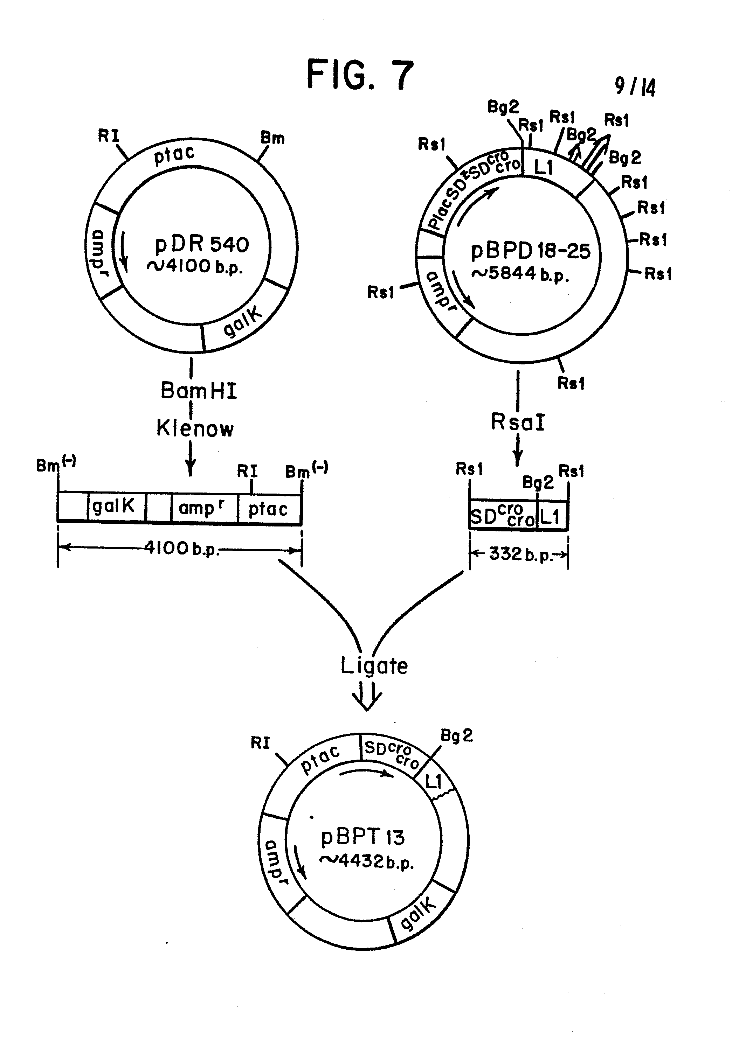

The expression vector pDR540 (about 4100 bp) contains the $amp^r$ (ß-lactamase) gene, a hybrid trp promoter/lac operator (Ptac), the lac ribosome binding site ($SD^{lac}$) and the galK (galactose kinase) gene (see FIG. 7). This plasmid was obtained commercially from P.L. Biochemicals, Inc., Milwaukee, WI.

pDR540 had a unique BamHI site immediately downstream from the $SD^{lac}$. When a DNA fragment, containing a gene with its ATG appropriately close to the $SD^{lac}$ proximal end, is inserted into the BamHI site, the gene is expressed at high levels by the hybrid tac promoter.

Plasmid pDR540 was isolated as per Section 6.1.1. and completely digested with restriction endonuclease BamHI. The BamHI cohesive termini were rendered blunt-ended by filling in with the Klenow fragment of E. coli DNA polymerase I as per Section 6.1.4. This resulted in elimination of the BamHI cohesive termini and the creation of blunt ends on the DNA fragment. The linearized, blunt ended plasmid was isolated by agarose gel electrophoresis and purified from LTA gel as described in Section 6.1.7.

Plasmid pBPD18 (FIG. 7) was isolated as described in Section 6.1.1. and completely digested with restriction endonuclease RsaI.

After agarose gel electrophoresis, a DNA fragment of about 332 bp was purified frm LTA as described in Section 6.1.7. This fragment contained a RsaI blunt end,

the SD$\underline{\text{cro}}$, the ATG and the amino-terminal portion of the cro/L1 gene fusion, and a RsaI blunt end.

The DNA fragments from pBPD18-25 and pDR540 were then ligated in equal concentration at a final total DNA concentration of 25 ng/µℓ. Reaction conditions were as described in Section 6.1.6. The ligated DNA was used to transform E. coli K12, strain NF1829, as described in Section 6.1.1.

### 6.6.4. IDENTIFICATION OF TRANSFORMANTS CONTAINING THE TAC PROMOTER AND THE AMINO-TERMINAL PORTION OF CRO/L1

The linearized pDR540 DNA contained the complete ß-lactamase gene, which provide ampicillin resistance; therefore, transformants were selected for ampicillin resistance.

Plasmid DNA from ampicillin resistant isolates was purified as per Section 6.1.1., cleaved by restriction endonucleases EcoRI and BglII, and analyzed by agarose gel electrophoresis. One such isolate, designated pBPT13 (FIG. 7) produced two fragments of about 4000 bp and 432 bp which indicated that the 332 bp RsaI DNA fragment from pBPD18-25 had been inserted in the correct orientation.

### 6.6.5. RECONSTRUCTION OF THE L1 GENE-STEP 1

Because of the absence of a useful restriction endonuclease site within the portion of the L1 gene present in pBPT13, it was necessary to reconstruct the complete L1 gene in two steps.

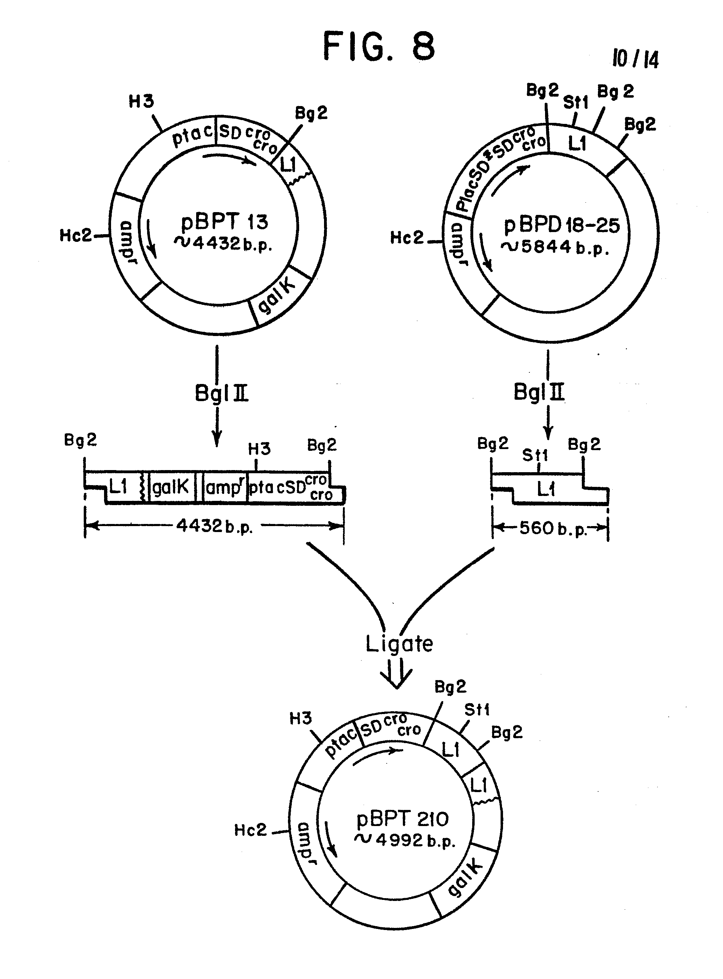

In the first step plasmid pBPT13 (see FIG. 8) was purified as described in Section 6.1.1. and completely digested with the restriction endonuclease BglII. The resulting linear plasmid DNA, containing BglII cohesive ends was isolated by agarose gel electrophoresis and purified from LTA gel as described in Section 6.1.7.

Plasmid pBPD18-25 (see FIG. 8) was purified as described in Section 6.1.1. and completely digested with the restriction endonuclease BglII. A fragment of about 560 bp, containing a portion of the L1 gene, including a StuI site and cohesive BglII ends, was isolated by agarose gel electrophoresis and purified from LTA gel as described in Section 6.1.7.

The DNA fragments from pBPD18-25 and pBPR13 were then ligated in equal concentration at a final total DNA concentration of 25 ng/µℓ. Reaction conditions were as described in Section 6.1.6. The ligated DNA was used to transform E. coli K12, strain NF1829, as described in Section 6.1.1.

### 6.6.6. IDENTIFICATION OF TRANSFORMANTS CONTAINING THE STUI SITE

The linearized pBPT13 DNA (Section 6.6.5.) contained the complete ß-lactamase gene, which provides ampicillin resistance; therefore, transformants were selected for ampicillin resistance.

Plasmid DNA from ampicillin resistant isolates was purified as per Section 6.1.1., cleaved by restriction endonucleases HindIII and StuI, and analyzed by agarose gel electrophoresis. One such isolate, designated pBPT210 (FIG. 8) produced two fragments of about 530 bp and 4462 bp, which indicated that the 530 bp BglII DNA fragment from pBPD18-25 (see Section 6.6.5.) had been inserted in the correct orientation.

### 6.6.7. RECONSTRUCTION OF THE L1 GENE-STEP 2

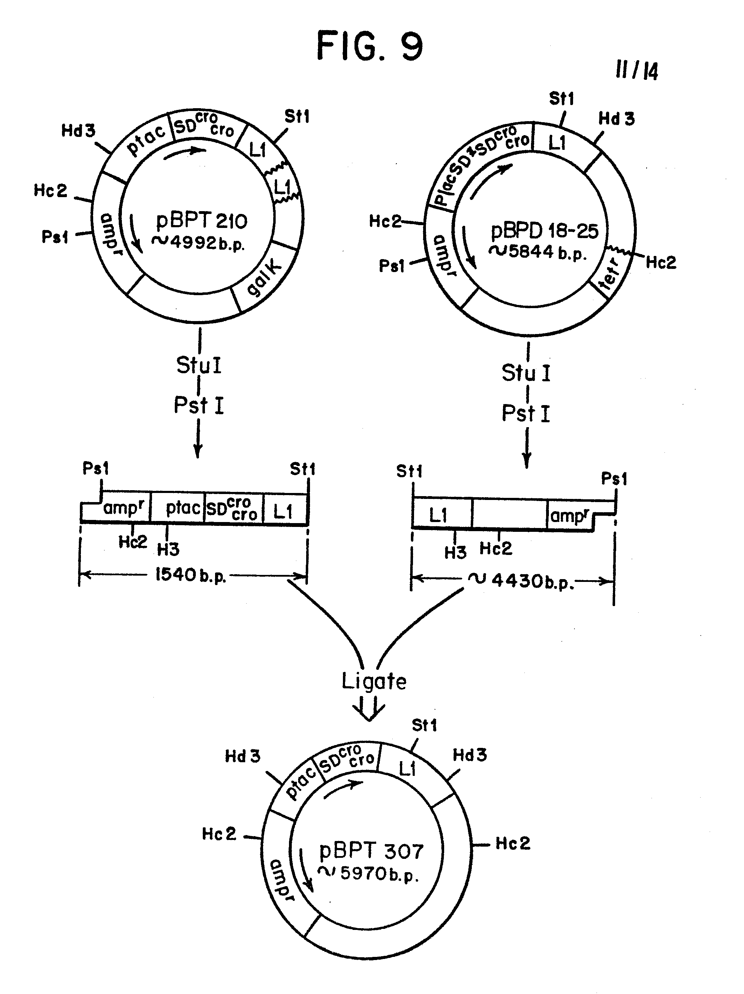

In the second step of the L1 gene reconstruction, plasmid pBPT210 (FIG. 9) was isolated as described in Section 6.1.1. and completely digested with restriction endonuclease StuI followed by complete digestion with PstI.

After agarose gel electrophoresis, a DNA fragment of about 1540 bp was purified from LTA as described in Section 6.1.7. This fragment contained a PstI cohesive end, the amino-terminal portion and all transcriptional and translational signals for the $amp^r$ gene, the tac transcriptional signals, the cro translational signals, the cro/L1 sequence and a StuI blunt end.

Plasmid pBPD18-25 (see FIG. 9) was purified as described in Section 6.1.1. and completely digested with the restriction endonucleases StuI and PstI. The resulting DNA fragment (about 4430 bp) containing a StuI blunt end, an internal portion of the L1 gene, a small portion of the $tet^r$ gene, the carboxy-terminal portion of the ß-lactamase gene ($amp^r$) and a PstI cohesive end was isolated by agarose gel electrophoresis and purified from LTA gel as described in Section 6.1.7.

The DNA fragments from pBPD18-25 and pBPT210 were then ligated in equal concentration at a final total DNA concentration of 25 ng/µℓ. Reaction conditions were as described in Section 6.1.6. The ligated DNA was used to transform E. coli K12, strain JS1060, as described in Section 6.1.1.

### 6.6.8. IDENTIFICATION OF TRANSFORMANTS EXPRESSING CRO/L1 FUSION PROTEIN

The PstI/StuI pBPT210 DNA fragment (Section 6.6.7.) contained the transcription and translational signals as well as the amino-terminal portion of the ß-lactamase gene, which provides ampicillin resistance. The StuI/PstI pBPD18-25 DNA fragment from Section 6.6.7. contained the portion of the ß-lactamase gene missing from the pBPT210 DNA fragment. When ligated the desired recombinant plasmid generated a complete ß-lactamase gene ($amp^r$); therefore, transformants were selected for ampicillin resistance.

Plasmid DNA from ampicillin resistant isolates was purified as per Section 6.1.1., cleaved by restriction endonucleases HindIII and HincII, and analyzed by agarose gel electrophoresis. One such isolate, designated pBPT307 (FIG. 9) produced four fragments of about 3254 bp, 1426 bp, 704 bp and 586 bp, which indicated that the cro/L1 gene had been completely reconstructed.

A total cell lysate was prepared from E. coli containing pBPT307. The proteins isolated from this cell lysate were analyzed by SDS polyacrylamide gel electrophoresis (SDS-PAGE). The isolate produced a large protein of the expected size, about 51,000 daltons molecular weight, while the controls did not.

SDS-PAGE also showed that E. coli containing pBPT307 produced about 35 mg of cro/L1 fusion protein per liter of culture; whereas, the analogous construction with the lac promoter (pBPD18) produced no detectable cro/L1 fusion protein (i.e., less than 0.6 mg per liter).

## 6.7. CLONING AND EXPRESSION OF THE L2 GENE

### 6.7.1. INSERTION OF THE L2 GENE INTO THE EXPRESSION VECTOR pUC9

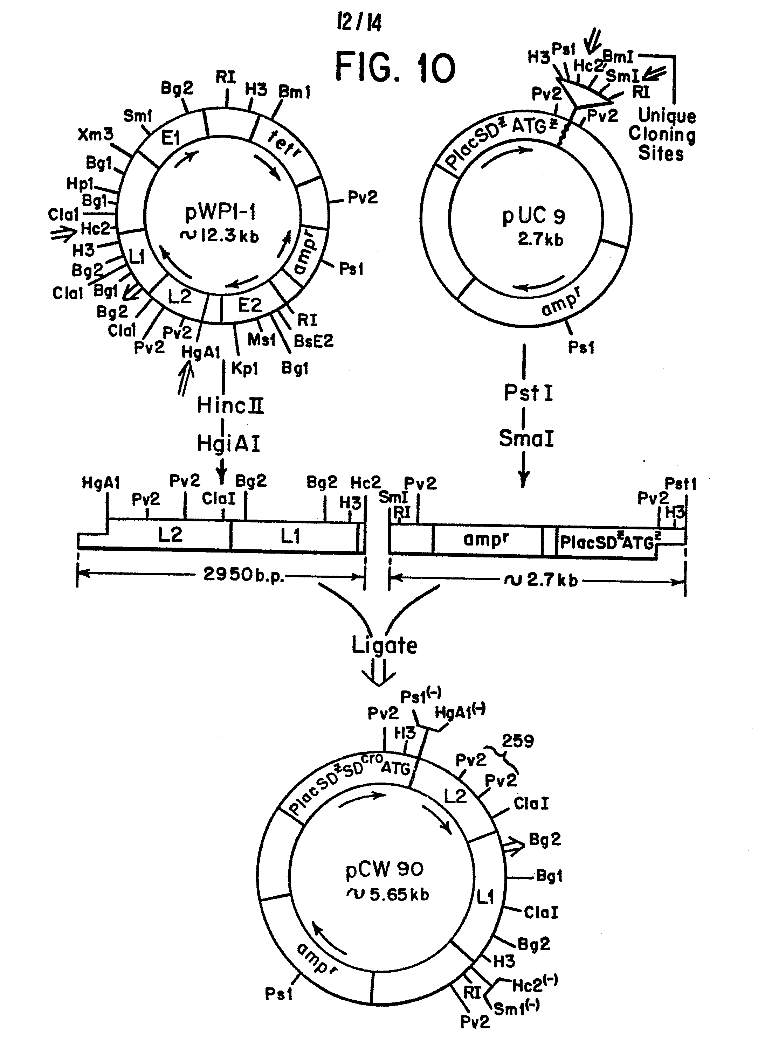

Plasmid pWP1-1 was purified as described in Section 6.1.1. and cleaved with the restriction endonucleases HincII and HgiAI (see FIG. 10).

After agarose gel electrophoresis, a DNA fragment of about 2950 bp was purified from LTA as per Section 6.1.7. This fragment contained an HgiAI cohesive end (which is complementary to a PstI cohesive end), the L2 gene without its initiation ATG (hereinafater referred to as the L2 gene), the L1 gene, and a HincII blunt end.

Plasmid pUC9 (Vieira and Messing, 1982, Gene 19:259-268) was purified as per Section 6.1.1. and cleaved with restriction endonucleases PstI (partial digestion) and SmaI (total digestion) (See FIG. 10). After agarose gel electrophoresis, a DNA fragment of about 2700 bp was purified from LTA as per Section 6.1.7. This fragment contained a SmaI blunt end, the ß-lactamase genes coding for ampicillin resistance, the lac transcription and translational signals, including the galactosidase ATG and a region coding for the amino-terminal portion of the ß-galactosidase protein, and a PstI cohesive end.

The isolated DNA fragments from pWP1-1 (HgiAI/HincII) and pUC9 (SmaI/PstI) were then ligated at 1:1 molar ratio at a final total DNA concentration of 30 ng/µℓ. Reaction conditions were as per Section 6.1.6. The ligated DNA was used to transform E. coli K12, strain MC1000.

### 6.7.2. IDENTIFICATION OF TRANSFORMANTS CONTAINING THE L2 GENE

The SmaI/PstI pUC9 fragment contained the complete β-lactamase gene; therefore, transformants were selected for ampicillin resistance.

Plasmid DNA from ampicillin resistant isolates was purified as per Section 6.1.1., cleaved by restriction endonuclease PvuII and analyzed by agarose gel electrophoresis. Restriction digests of plasmid obtained from one such isolate, designated pCW90, produced four fragments of about 2419 bp (the pUC9 portion of pCW90), 2203 bp, 770 bp and 259 bp.

### 6.7.3. INSERTION OF THE L2 GENE INTO EXPRESSION VECTOR pHK413

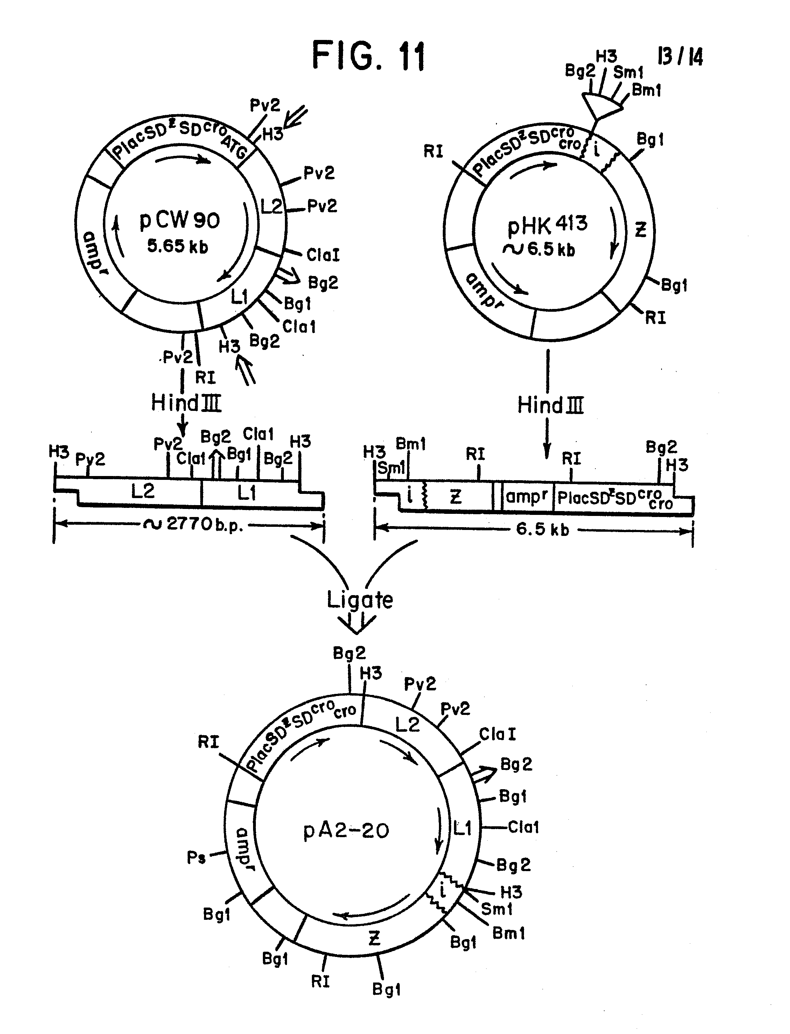

Plasmid pCW90 was purified as per Section 6.1.1. and completely digested with restriction endonuclease HindIII (see FIG. 11). After agarose gel electrophoresis, a DNA fragment of about 2770 bp was purified from LTA as per Section 6.1.7. This fragment contained the L2 gene, part of the L1 gene, and possessed HindIII cohesive ends on both termini (FIG. 11).

Plasmid pHK413 was purified as per Section 6.1.1. cleaved with restriction endonuclease HindIII, and purified from LTA after gel electrophoresis as per Section 6.1.7. (FIG. 11). This generated a single DNA fragment of about 6500 bp (6.5 kb) which contained two HindIII cohesive ends, the lac transcriptional signals, the cro translational signals, including an amino-terminal portion of the lambda cro gene, the amp$^r$ gene and the β-galactosidase gene (z-gene).

The DNA fragments from pCW90 and pHK413 were then ligated at 1:1 molar ratio at a final total DNA concentration of 25 ng/ml. Reaction conditions were as described in Section 6.1.6. The ligated DNA was used to transform E. coli K12, strain NF1829, as described in Section 6.1.1.

### 6.7.4. IDENTIFICATION OF TRANSFORMANTS CONTAINING THE L2 GENE

The pHK413 DNA fragment contained a complete β-lactamase gene providing ampicillin resistance; therefore, transformants were selected for ampicillin resistance.

Plasmid DNA obtained from ampicillin resistant isolates was purified as per Section 6.1.1., cleaved by restriction endonuclease BglI and analyzed by agarose gel electrophoresis. Restriction digests of plasmid obtained from one such isolate, designated pA2-20, produced five DNA fragments of about 3429 bp, 2142 bp, 2121 bp, 872 bp and 661 bp.

### 6.8. PREPARATION OF pFU41 WHICH DIRECTS THE PRODUCTION OF CRO/L2/β-GALACTOSIDASE FUSION PROTEIN

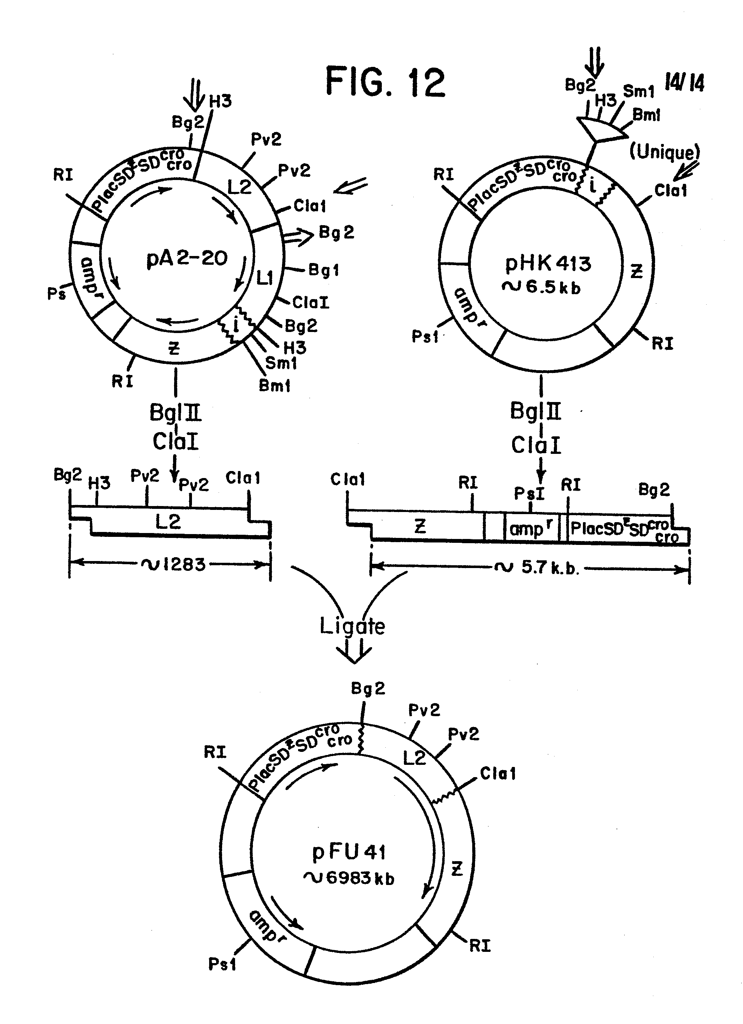

As explained in the following subsections, the L2 gene was isolated from pA2-20 and inserted into pHK413 resulting in pFU41 in which the L2 protein coding sequence is in the same translational reading frame as the pHK413 cro ATG and the z-gene (see FIG. 12).

### 6.8.1. PREPARATION AND LIGATION OF DNA FRAGMENTS

Plasmid pA2-20 was purified as per Section 6.1.1. and completely digested with restriction endonucleases

BglII and ClaI.  After agarose gel electrophoresis, a DNA fragment of about 1283 bp, containing a BglII cohesive end, the L2 gene and a ClaI cohesive end, was purified from LTA as per Section 6.1.7.

Plasmid pHK413 was purified as per Section 6.1.1. and completely digested with BglII and ClaI.  After agarose gel electrophoresis, a DNA fragment of about 5700 bp, containing a ClaI cohesive end, the z-gene, the amp$^r$ gene, the lac tanscriptional signals, the cro translational signals, including an amino-terminal portion of the cro gene, and a BglII cohesive end, was purified from LTA as per Section 6.1.7.

The DNA fragments from pA2-20 (BglII/ClaI) and pHK413 (ClaI/BglII) were ligated at 1:1 molar ratio at a final total DNA concentration of 18 ng/µℓ as per Section 6.1.6.  The ligated DNA was used to transform E. coli K12, strain NF1829, as per Section 6.2.4.

### 6.8.2.  IDENTIFICATION OF TRANSFORMANTS CONTAINING CRO/L2/ß-GALACTOSIDASE DNA

The ClaI/BglII pHK413 DNA fragment isolated above contained the ß-lactamase gene, providing ampicillin resistance; therefore, transformants were selected for ampicillin resistance.

Plasmid DNA from ampicillin resistant isolates was purified as per Section 6.1.1., cleaved by restriction endonucleases BglII and ClaI and analyzed by agarose gel electrophoresis.  Restriction digests of plasmid obtained from one such isolate, designated pFU41, produced two DNA fragments of about 1283 bp and 5700 bp.

### 6.8.3.   IDENTIFICATION OF CLONES EXPRESSING CRO/L2/ß-GALACTOSIDASE FUSION PROTEIN

Total cell lysates were prepared from a culture of pFU41 transformed E. coli and the cellular proteins of induced and uninduced transformants were analyzed by SDS polyacrylamide gel electrophoresis (SDS-PAGE). Induction was accomplished by the addition of 1 mM IPTG to the growth media. Induced pFU41 transformed E. coli produced a large protein of the expected molecular weight (i.e., as predicted based upon the length of the cro/L2/ß-galactosidase DNA sequence), approximately 150,000 daltons. The uninduced E. coli transformed with pFU41, as well as the appropriate controls, did not produce fusion proteins.

### 6.9.   EVALUATION OF BOVINE PAPILLOMAVIRUS CAPSID FUSION PROTEINS

### 6.9.1.   FUSION PROTEIN IMMUNOREACTS WITH ANTI-BPV-1 SERUM

The cro/L1/ß-galactosidase fusion protein produced by pC2V1 transformants was separated from other cellular proteins by SDS-PAGE. The fusion proteins were eluted from the gels and concentrated by the procedure described in Section 6.1.8. The purified cro/L1/ß-galactosidase fusion protein of pC2V1 reacted positively with antibody directed against whole BPV-1 when assayed by the enzyme linked immunosorbent assay (ELISA; Voller, A. et al., in Manual of Clinical Immunology, 2d Ed., Rose and Friedman, Eds., ASM, Washington, DC (1980): 359-371). Details are described below.

Rabbit antiserum directed against whole BPV-1 was produced as follows: 0.6 mℓ of purified BPV-1 virus (see Section 6.2.1) at a total protein concentration of 1 mg/mℓ (determined by a colorimetric assay, Lowry et al., 1951, J. Biol. Chem. 193:265-275) was combined with 0.9 mℓ complete Freund's adjuvant. After vortexing, the mixture was gently shaken overnight in order to form an emulsion, and 0.75 mℓ (approximately 300 μg BPV-1) was injected intramuscularly into a New Zealand rabbit. After 28 days the rabbit was boosted with 0.25 mℓ of BPV-1 (100 μg BPV-1) prepared as above suspended in incomplete Freund's adjuvant. Serum was collected 7 days after the boost and analyzed by ELISA using purified BPV-1 as antigen. The boosts were repeated and serum collected and analyzed two more times until the animal demonstrated a high titer of antibody directed against BPV-1 (anti-BPV-1).

The enzyme linked immunosorbent assay, or ELISA, was performed using as antigen the cro/L1/ß-galactosidase fusion protein produced by pC2V1 transformants (or pC2V1 fusion protein) as follows: the pC2V1 fusion protein was diluted to 50 μg/mℓ in fresh carbonate buffer (stock carbonate buffer: 0.635 g $Na_2CO_3$, 0.504 g $NaHCO_3$ in 250 mℓ $H_2O$). Then 100 μℓ aliquots of the pC2V1 antigen solution was added to 6 wells of a microtiter plate which was incubated overnight at 37°C to allow the pC2V1 fusion protein to adsorb to the wells. The wells were then washed with Tween Buffer: 146.1 g NaCl, 200 mℓ 0.5 M $Na_2HPO_4$, 25 mℓ Tween 20 (polyoxyethylene sorbitan monolaurate) and distilled water to 1000 mℓ total volume. Pre-immune serum, and rabbit anti-BPV-1 serum were each serially diluted (1:10, 1:100, 1:1000) with Tween buffer. Each of the 6 microtiter wells received a 50 μℓ aliquot of one serum dilution. After incubating at room temperature for 30 minutes with shaking a 100 μℓ aliquot of peroxidase labeled goat anti-rabbit Ig solution was added and the wells were incubated at room temperature for 15 minutes with shaking. The wells were washed three times with Tween buffer and 100 μℓ freshly made OPD indicator was added to each of the six wells:  5 mg OPD (Orthophenyldiamine) in 12.5 mℓ OPD buffer (25 mℓ 0.1 M citrate, 9.5 mℓ 0.5 M $Na_2HPO_4$ and 65.5 mℓ $H_2O$) plus 5 μℓ 30% $H_2O$.  After a 15 minute incubation at room temperature, a 100 μℓ aliquot of 2.5 M $H_2SO_4$ was added to each well.  The color intensity of the wells was recorded and photographed.

### 6.9.2.  ANTISERA DIRECTED AGAINST FUSION PROTEINS IMMUNOREACT WITH BPV-1 PROTEINS

Rabbit antisera were generated against each fusion protein as follows:  the cro/L1/ß-galactosidase fusion protein produced by pC2V1 transformants and the cro/L2/ß-galactosidase fusion protein produced by pFU41 transformants were separated from other cellular proteins by SDS-PAGE.  After SDS-PAGE, the proteins were visualized by staining the outer lanes with coomassie blue dye; then the fusion proteins were sliced from the gels (as described in Section 6.1.8).  Each gel slice was immersed in liquid nitrogen, ground to a powder and then each was suspended in an equal volume of Freund's complete adjuvant.  After thorough mixing, the solutions were injected subcutaneously into two New Zealand rabbits.  Each rabbit was injected with 100 to 300 μg protein.  After 28 days, the rabbits were boosted with the same fusion protein suspended in incomplete Freund's adjuvant.  The animals were boosted 2 more times.  The sera (hereinafter referred to as anti-pC2V1 serum and anti-pFU41 serum) collected 63 days after the initial injection were analyzed for immunoreactivity with BPV-1 proteins by ELISA assays (described in 6.9.1) using BPV-1 proteins as antigen and by immunoprecipitation of radiolabeled BPV-1 proteins (described below).

BPV-1 proteins were radiolabeled with $^{125}$I using the solid phase lactoperoxidase-glucose oxidase system for radioiodination (Enzymobeads, BioRad Laboratories, Richmond, CA) in a final reaction volume of 200 µℓ as follows: BPV-1 was isolated from wart tissue and purified in a CsCl gradient as previously described (final preparation containing approximately 2 mg/mℓ total protein). The following was added to 50 µℓ Enzymobeads (which were reconstituted as per instructions) in a 1.5 mℓ microcentrifuge tube: 6 µℓ of 5 M NaCl (i.e., 150 mM final concentration), 50 µℓ of 0.2 M sodium phosphate (pH 7.2), and 10 µℓ $^{125}$I (100 mCi/mℓ, New England Nuclear, Boston, MA), 10 µℓ of the purified BPV-1, and distilled water to a final volume of 170 µℓ. To initiate the reaction 30 µℓ of 2% glucose (i.e., 1% ß-D-glucose and 1% alpha-D-glucose prepared one day in advance) was added to the Enzymobead reaction vessel. The reaction mixture was incubated for 1 hour at 30°C with intermittant shaking. The solid-phase enzymo beads were pelleted by centrifugation in a microcentrifuge (approximately 15,000 x g for 1 minute. The supernatant was applied to a 20 mℓ column comprising G-50 Sephadex (a bead-formed gel consisting of dextran cross-linked with epichlorohydrin (Pharmacia Fine Chemicals, Piscataway, NJ) in PBS (phosphate buffered saline) and 100 mg/mℓ BSA (bovine serum albumin). A fast moving peak of incorporated $^{125}$I separated from the unincorporated $^{125}$I. The $^{125}$I-labeled BPV-1 in the fast moving peak was used for immunoprecipitation analysis of the rabbit antisera directed against each fusion protein as follows:

A 10 µℓ aliquot of $^{125}$I-BPV-1 was mixed with 0.5 mℓ 20% SDS (SDS final concentration of 1%) and was incubated at 37°C for 30 minutes. Then 1 mℓ of RIPA buffer (150 mM NaCl, 10 mM Tris-HCl, pH 7.2, 1%

Triton X-100, 1% deoxycholate and 1% SDS) was added to the mixture which was then distributed into microcentrifuge tubes in 50 mℓ aliquots. Each antiserum tested (anti-pC2V1, anti-pFU41, and pre-immune sera as controls) was added to an individual aliquot of $^{125}$I-BPV-1 prepared as above and incubated at 4°C for 60 minutes.

During the 60 minute incubation period a 10% suspension of protein-A containing Staphylococcus aureus cells was washed twice with B buffer (120 mM NaCl, 50 mM Tris-HCl (pH 8.0), 0.5% NP-40) once with B buffer containing 0.5 M LiCl$_2$, and once more with B buffer without LiCl$_2$. The washed S. aureus was resuspended in B buffer to its original volume and ovalbumin was added to a final concentration of 1 mg/mℓ.

The washed S. aureus was added to each antiserum mixture in an amount equal to 5 times the volume of antiserum used and the mixture was incubated at 4°C for 20 minutes. The immune complexes were pelleted by centrifugation in a microcentrifuge (15,000 x g) and washed with B buffer containing 0.5 M LiCl$_2$, followed by B buffer.

The final pellets were resuspended in gel sample buffer consisting of 10% β-ME, 0.14 M Tris-HCl (pH 6.8), 22.4% glycerol, 6% SDS and 0.01% bromophenol blue and boiled for one minute. After removal of insoluble debris by centrifugation in a microcentrifuge, the material was electrophoresed in SDS polyacrylamide gels containing 10% polyacrylamide (Laemmli, 1970, Nature 227:680). The gels were prepared for fluorography by soaking in sodium salicylate (Chamberlain, 1979, Anal. BioChem 98:132) and the dried gels were exposed to Dupont Cronex 4 X-ray film at -70°C. Results of fluorography demonstrated that each rabbit antiserum immunoprecipitated the BPV-1 structrual proteins while the pre-immune sera did not. In fact, both antisera (anti-pC2V1 and pFU41) reacted positively against BPV-1 as demonstrated by both ELISA and the immune precipitation described above.

### 6.9.3. ANTISERA DIRECTED AGAINST FUSION PROTEINS INHIBIT CELL TRANSFORMATION BY BPV-1 IN VITRO

Both antibodies prevented in vitro cell transformation by BPV-1 in an assay developed by Dvoretzky et al. (1980, Virol. 103: 369-375). This quantitative tissue culture assay is based upon the fact that BPV-1 or BPV-2 induce distinct foci of morphologically transformed cells in vitro in two mouse cell lines (NIH 3T3 and C127). Dvoretzky et al. suggest that a single particle is required for focus induction. The morphologically transformed cells contain BPV DNA sequences, grow in low serum and in soft agar and are tumorigenic in nude mice. Preincubation of bovine papilloma extracts with rabbit anti-BPV-1 serum neutralizes virus infectivity and is reflected by a loss of transforming ability (e.g., a decrease in the number of foci formed).

Using the procedure described below, preincubation of bovine papilloma extracts with rabbit anti-fusion protein serum (anti-pC2V1 or anti-pFU41 prepared in Section 6.9.2) resulted in a reduction in the number of foci induced in C127 cells.

The supernatant of a preparation of crude virus (bovine papilloma extract containing approximately $3 \times 10^5$ focus forming units/ml, FFU/ml) was frozen in methanol/dry ice, thawed and sonicated three times for 15 seconds each in 50 mM Tris-HCl (pH 7.2), 100 mM NaCl. The resulting mixture was diluted 1:100 and divided into 3 ml aliquots.

Each aliquot received one of the following: no sera (control); 60 µℓ undiluted pre-immune serum; 60 µℓ of undiluted anti-fusion protein serum (anti-pC2V1 serum or anti-pFU41 serum); 60 µℓ of anti-fusion protein serum diluted 1:10; 60 µℓ of anti-fusion protein serum diluted 1:100. After mixing by vortexing for 10 seconds the papilloma extracts/antiserum mixtures were incubated at 37°C for 1 hour.

C127 cells were grown at 37°C in the following growth medium: Dulbecco's modified Eagle Media supplemented with 10% fetal calf serum and with penicillin and streptomycin (100 µg/mℓ each). Each 60 mm petri dish had been seeded with approximately $10^5$ cells on the previous day. The growth medium was removed and each plate received 1 mℓ of one of the papilloma extract/antiserum mixtures. The plates were incubated at 37°C for 1 hour with 10% $CO_2$ humidified air. During this time, the plates were tilted every 20 minutes. Then 10 mℓ of pre-warmed growth medium was added to each plate. The medium was replenished on the following day and then three times a week for 16 days. The plates were examined for foci formation, fixed, stained and the foci were counted. Virus neutralization by anti-pC2V1 or anti-pFU41 was indicated by a reduction in the number of foci formed in cells treated with mixtures of papilloma extract and rabbit anti-fusion protein serum (see Table I).

## TABLE I

### Papillomavirus Neutralization by Anti-Fusion Protein Sera

| | Number of Foci[1] | | |
|---|---|---|---|
| | Undiluted Serum | Diluted Serum | |
| Rabbit Serum | | $10^{-1}$ | $10^{-2}$ |
| Pre-immune | 137 | ND | ND |
| Anti-pC2V1 | 0 | 36 | 108 |
| Anti-pFU41 | 0 | 36 | 94 |

[1] C127 cells were treated with papilloma extracts preincubated with the rabbit sera indicated. See text for full explanation.

### 6.9.4. VACCINATION OF CATTLE WITH CRO/L1/ß-GALACTOSIDASE FUSION PROTEIN

Cro/L1/ß-galactosidase fusion protein was purified from E. coli containing pC2V1 as described in Section 5.7.

Whole cell preparations of E. coli containing pC2V1 were obtained from stationary cultures, heat treated at 65°C for 30 minutes.

Both purified fusion protein and whole cell preparation were further inactivated with BEI (Section 6.1.9).

0133123

Three month old calves were vaccinated as indicated in Table II. Three animals were vaccinated intramuscularly for each experiment and each animal was given an initial injection and an equivalent boost thirty-one days later.

TABLE II

BPV-1 Formulations

| Experiment | Product/ Injection | Adjuvant/ Injection | Final Volume/ Injection |
|---|---|---|---|
| A (initial) | 20 mg fusion protein | 1.2 mℓ complete Freund's[1] | 2.4 mℓ |
| (boost) | 20 mg fusion protein | 1.2 mℓ incomplete Freund's[1] | 2.4 mℓ |
| B | 20 mg fusion protein | 0.8 mℓ Rehsorptar[2] | 2.0 mℓ |
| C | 20 mg fusion protein | none | 1.2 mℓ |
| D | 10 mg fusion protein in 3 mℓ whole cells | 1.25 mℓ Rehsorptar[2] | 4.25 mℓ |
| E | none | none | none |

[1] Difco Labs, Detroit, MI

[2] Reheis Chemical Co., Berkely Heights, NJ

All animals were challenged 20 days after the boost by injecting 0.05 mℓ of BPV, purified as in Section 6.2.1., intradermally at two sites on the neck.

The animals were examined forty-four days after the challenge. As shown in Table III, only two of twelve (17%) vaccinated animals developed warts while all three (100%) non-vaccinated animals developed warts. Thus, vaccination of cattle with cro/L1/ß-galactosidase fusion protein inhibited wart formation by BPV-1.

---

TABLE III

Inhibition of Wart Formation

| Experiment (see Table II) | # of animals with warts/<br># of animals challenged |
|---|---|
| A | 1/3 |
| B | 1/3 |
| C | 0/3 |
| D | 0/3 |
| E | 3/3 |

---

7. DEPOSIT OF MICROORGANISMS

The following E. coli strains carrying the listed plasmids have been deposited with the Agricultural Research Culture Collection (NRRL) Peoria, IL, and have been assigned the following accession numbers:

| E. Coli Strain | Plasmid | Accession Numbers |
|---|---|---|
| K12, NF1829 | pBPD18 | NRRL B-15520 |
| K12, NF1829 | pFU41 | NRRL B-15521 |
| K12, NF1829 | pCW90 | NRRL B-15522 |
| K12, NF1829 | pC2V1 | NRRL B-15523 |
| K12, JS1060 | pBPT307 | NRRL B-15803 |

The present invention is not to be limited in scope by the microorganisms deposited, since the deposited embodiment is intended as single illustration of one aspect of the invention and any microorganisms which are functionally equivalent are within the scope of this invention. Indeed, various modifications of the invention in addition to those shown and described herein will become apparent to those skilled in the art from the foregoing description and accompanying drawings. Such modifications are intended to fall within the scope of the appended claim.

It is also to be understood that all base pair sizes given for nucleotides are approximate and are used for purpose of description.

WE CLAIM:

1.    A process for producing a polypeptide having an immunoreactive and antigenic determinant of a papillomavirus protein, comprising:

        (a)   culturing a unicellular organism containing a recombinant vector comprising a DNA sequence coding for a polypeptide having an immunoreactive and antigenic determinant of a papillomavirus protein and capable of being replicated, transcribed and translated in said unicellular organism; and

        (b)   isolating said polypeptide from the culture.

2.    The process according to claim 1, wherein said polypeptide is immunogenic.

3.    The process according to claim 1, wherein the recombinant vector was introduced into the unicellular organism by transformation.

4.    The process according to claim 1, wherein the recombinant vector was introduced into the unicellular organism by transduction.

5.    The process according to claim 1, wherein the recombinant vector was introduced into the unicellular organism by transfection.

6.    The process according to claim 1, wherein the information contained in the DNA sequence is obtained from a papillomavirus genome.

7.    The process according to claim 6, wherein the information contained in the DNA sequence is obtained from bovine papillomavirus.

8.    The process according to claim 1, wherein the information contained in the DNA sequence is obtained by isolating mRNA coding for a papillomavirus protein and using reverse transcriptase to construct said DNA sequence having one strand complementary to the isolated mRNA.

9.    The process according to claim 1, wherein the DNA sequence is obtained from a DNA vector containing a papillomavirus DNA sequence.

10.    The process according to claim 1, wherein the DNA sequence codes for a structural protein of a papillomavirus.

11.    The process according to claim 1, wherein the DNA sequence codes for a structural protein of a bovine papillomavirus.

12.    The process according to claim 1, wherein the unicellular organism is a eucaryotic organism.

13.    The process according to claim 1, wherein the unicellular organism is a procaryotic organism.

14.    The process according to claim 13, wherein the procaryotic organism is _Escherichia coli_.

15.    The process according to claim 14, wherein the _Escherichia coli_ has NRRL accession No. B-15520 or a mutant, recombinant or genetically engineered derivative thereof.

16. The process according to claim 14, wherein the *Escherichia* coli has NRRL accession No. B-15521 or a mutant, recombinant or genetically engineered derivative derivative thereof.

17. The process according to claim 14, wherein the *Escherichia* coli has NRRL accession No. B-15522 or a mutant, recombinant or genetically engineered derivative thereof.

18. The process according to claim 14, wherein the *Escherichia* coli has NRRL accession No. B-15523 or a mutant, recombinant or genetically engineered derivative thereof.

19. The process according to claim 14, wherein the *Escherichia* coli has NRRL accession No. B-15803 or a mutant, recombinant or genetically engineered derivative thereof.

20. A process for preparing a unicellular organism having a DNA sequence coding for a polypeptide having an immunoreactive and antigenic determinant of a Papillomavirus protein, comprising: introducing a recombinant vector into a unicellular organism, said recombinant vector comprising a DNA sequence coding for a polypeptide having an immunoreactive and antigenic determinant of a papillomavirus protein and capable of being replicated, transcribed and translated in the unicellular organism.

21. A recombinant DNA vector, comprising: a DNA sequence coding for an immunoreactive and antigenic determinant of a papillomavirus protein.

22. The recombinant DNA vector according to claim 21, wherein the Papillomavirus is bovine papillomavirus.

23. The recombinant DNA vector according to claim 22 wherein the DNA sequence codes for a bovine papillomavirus structural protein L1 as depicted in FIG. 3, or any portion thereof.

24. The recombinant DNA vector according to claim 22 wherein the DNA sequence codes for a bovine papillomavirus structural protein L2 as depicted in FIG. 3, or any portion thereof.

25. A recombinant DNA vector of claim 22, wherein said DNA sequence is under the control of expression control elements.

26. A recombinant vector according to claim 25, wherein the vector is pBPD18, or a mutant, recombinant, or genetically engineered derivative thereof.

27. A recombinant vector according to claim 25, wherein the vector is pCW90, or a mutant, recombinant, or genetically engineered derivative thereof.

28. A recombinant vector according to claim 25, wherein the vector is pBPT307, or a mutant, recombinant, or genetically engineered derivative thereof.

29. A recombinant DNA vector of claim 22, further comprising the essential portions of the pBR322 replicon.

30. A recombinant DNA vector of claim 22, wherein said DNA sequence is inserted in the correct reading frame to a second DNA sequence coding for a protein.

31. A recombinant vector according to claim 30, wherein the vector is pC2V1, or a mutant, recombinant, or genetically engineered derivative thereof.

32. A recombinant vector according to claim 30, wherein the vector is pFU41, or a mutant, recombinant, or genetically engineered derivative thereof.

33. A unicellular organism containing the recombinant DNA vector of claim 21.

34. A unicellular organism containing the recombinant DNA vector of claim 22.

35. A unicellular organism containing the recombinant DNA vector of claim 23.

36. A unicellular organism containing the recombinant DNA vector of claim 24.

37. A unicellular organism containing the recombinant DNA vector of claim 25.

38. A unicellular organism containing the recombinant DNA vector of claim 29.

39. A unicellular organism containing the recombinant DNA vector of claim 30.

40. An _Escherichia_ _coli_ bacterium containing the recombinant DNA vector of claim 22.

41. An _Escherichia_ _coli_ bacterium containing the recombinant DNA vector of claim 23.

42. An _Escherichia_ _coli_ bacterium containing the recombinant DNA vector of claim 24.

43. An _Escherichia_ _coli_ bacterium containing the recombinant DNA vector of claim 25.

44. An _Escherichia_ _coli_ bacterium containing the recombinant DNA vector of claim 29.

45. An _Escherichia_ _coli_ bacterium containing the recombinant DNA vector of claim 30.

46. An _Escherichia_ _coli_ bacterium of claim 41 deposited with the NRRL and assigned accession No. B-15520, or a mutant, recombinant, or genetically engineered derivative thereof.

47. An _Escherichia_ _coli_ bacterium of claim 41 deposited with the NRRL and assigned accession No. B-15523, or a mutant, recombinant, or genetically engineered derivative thereof.

48. An _Escherichia_ _coli_ bacterium of claim 41 deposited with the NRRL and assigned accession No. B-15803, or a mutant, recombinant, or genetically engineered derivative thereof.

49. An _Escherichia_ _coli_ bacterium of claim 42 deposited with the NRRL and assigned accession No. B-15521, or a mutant, recombinant, or genetically engineered derivative thereof.

50. An _Escherichia_ _coli_ bacterium of claim 42 deposited with the NRRL and assigned accession No. B-15522, or a mutant, recombinant, or genetically engineered derivative thereof.

51. A polypeptide produced according to the process of claim 2.

52. A polypeptide having an immunoreactive and antigenic determinant of a bovine papillomavirus produced by the unicellular organism of claim 37.

53. A polypeptide having an immunoreactive and antigenic determinant of a bovine papillomavirus produced by _Escherichia_ _coli_ of claim 43.

54. A polypeptide having an immunoreactive and antigenic determinant of a bovine papillomavirus produced by _Escherichia_ _coli_ of claim 46.

55. A polypeptide having an immunoreactive and antigenic determinant of a bovine papillomavirus produced by _Escherichia_ _coli_ of claim 47.

56. A polypeptide having an immunoreactive and antigenic determinant of a bovine papillomavirus produced by _Escherichia_ _coli_ of claim 48.

57. A polypeptide having an immunoreactive and antigenic determinant of a bovine papillomavirus produced by _Escherichia_ _coli_ of claim 49.

58. A polypeptide having an immunoreactive and antigenic determinant of a bovine papillomavirus produced by _Escherichia_ _coli_ of claim 50.

59. A polypeptide of claim 51 which, when used as an immunogen, is capable of eliciting antibodies that neutralize virus infectivity.

60. A vaccine formulation comprising the polypeptide of claim 59 further comprising a compatible pharmaceutical carrier therefor.

61. A vaccine formulation comprising a lysate of the unicellular organism of claim 37.

62. The vaccine formulation according to claim 61 further comprising a compatible pharmaceutical carrier therefor.

63. A vaccine formulation comprising a lysate of the Escherichia coli of claim 43.

64. The vaccine formulation according to claim 63 further comprising a compatible pharmaceutical carrier therefor.

65. A vaccine formulation comprising a lysate of the Escherichia coli of claim 47.

66. The vaccine formulation according to claim 65 further comprising a compatible pharmaceutical carrier therefor.

67. A vaccine formulation comprising a lysate of the Escherichia coli of claim 48.

68. The vaccine formulation according to claim 67 further comprising a compatible pharmaceutical carrier therefor.

69. A vaccine formulation comprising a lysate of the _Escherichia coli_ of claim 49.

70. The vaccine formulation according to claim 69 further comprising a compatible pharmaceutical carrier therefor.

71. A vaccine formulation comprising a lysate of the _Escherichia coli_ of claim 50.

72. The vaccine formulation according to claim 71 further comprising a compatible pharmaceutical carrier therefor.

73. A vaccine formulation comprising a cell extract of the unicellular organism of claim 37.

74. The vaccine formulation according to claim 73 further comprising a compatible pharmaceutical carrier therefor.

75. A vaccine formulation comprising a cell extract of the _Escherichia coli_ of claim 43.

76. The vaccine formulation according to claim 75 further comprising a compatible pharmaceutical carrier therefor.

77. A vaccine formulation comprising a cell extract of the _Escherichia coli_ of claim 47.

78. The vaccine formulation according to claim 77 further comprising a compatible pharmaceutical carrier therefor.

79. A vaccine formulation comprising a cell extract of the Escherichia coli of claim 48.

80. The vaccine formulation according to claim 79 further comprising a compatible pharmaceutical carrier therefor.

81. A vaccine formulation comprising a cell extract of the Escherichia coli of claim 49.

82. The vaccine formulation according to claim 81 further comprising a compatible pharmaceutical carrier therefor.

83. A vaccine formulation comprising a cell extract of the Escherichia coli of claim 50.

84. The vaccine formulation according to claim 83 further comprising a compatible pharmaceutical carrier therefor.

85. An aggregate protein comprising an aggregate protein derived from the unicellular organism of claim 37.

86. An aggregate protein comprising an aggregate protein derived from the unicellular organism of claim 43.

87. An aggregate protein comprising an aggregate protein derived from the Escherichia coli of claim 47.

88. An aggregate protein comprising an aggregate protein derived from the Escherichia coli of claim 48.

89. An aggregate protein comprising an aggregate protein derived from the Escherichia coli of claim 49.

90. An aggregate protein comprising an aggregate protein derived from the _Escherichia_ _coli_ of claim 50.

91. A vaccine formulation comprising the aggregate protein of claim 85.

92. The vaccine formulation according to claim 91 further comprising a compatible pharmaceutical carrier therefor.

93. A vaccine formulation comprising the aggregate protein of claim 86.

94. The vaccine formulation according to claim 93 further comprising a compatible pharmaceutical carrier therefor.

95. A vaccine formulation comprising the aggregate protein of claim 87.

96. The vaccine formulation according to claim 95 further comprising a compatible pharmaceutical carrier therefor.

97. A vaccine formulation comprising the aggregate protein of claim 88.

98. The vaccine formulation according to claim 97 further comprising a compatible pharmaceutical carrier therefor.

99. A vaccine formulation comprising the aggregate protein of claim 89.

100. The vaccine formulation according to claim 99 further comprising a compatible pharmaceutical carrier therefor.

101. A vaccine formulation comprising the aggregate protein of claim 90.

102. The vaccine formulation according to claim 101 further comprising a compatible pharmaceutical carrier therefor.

103. A polypeptide comprising a substantially pure polypeptide having an immunoreactive and antigenic determinant of a papillomavirus protein.

104. A vaccine formulation comprising the polypeptide of claim 103.

105. The vaccine according to claim 104 further comprising a compatible pharmaceutical carrier therefor.

106. The polypeptide according to claim 103 in which the polypeptide is fused to a second polypeptide.

107. A vaccine formulation comprising the polypeptide of claim 106.

108. The vaccine according to claim 107 further comprising a compatible pharmaceutical carrier therefor.

109. The polypeptide according to claim 103 in which the polypeptide is capable of forming aggregates.

110. A vaccine formulation comprising the polypeptide of claim 109.

111. The vaccine according to claim 110 further comprising a compatible pharmaceutical carrier therefor.

112. A vaccine formulation comprising inactivated whole cells of the unicellular organism of claim 37.

113. A vaccine formulation comprising inactivated whole cells of the Escherichia coli of claim 43.

114. A vaccine formulation comprising inactivated whole cells of the Escherichia coli of claim 47.

115. A vaccine formulation comprising inactivated whole cells of the Escherichia coli of claim 48.

116. A vaccine formulation comprising inactivated whole cells of the Escherichia coli of claim 49.

117. A vaccine formulation comprising inactivated whole cells of the Escherichia coli of claim 50.

# FIG. 1A

1/14

# FIG. 1B

# FIG. 2

pFU4I,pCW90

L2→ (HgIAI)

ATG AGT GCA CGA AAA AGA GTA AAA CGT GCC AGT GCC TAT GAC CTG TAC AGG ACA TGC AAG  60
1   MET SER ALA PRO LYS ARG VAL LYS ARG ALA SER ALA TYR ASP LEU TYR ARG THR CYS LYS

CAA GCG GGC ACA TGT CCA CCA GAT GTG ATA CCA AAG GTA GAA GGA GAT ACT ATA GCA GAT  120
21  GLN ALA GLY THR CYS PRO PRO ASP VAL ILE PRO LYS VAL GLU GLY ASP THR ILE ALA ASP

AAA ATT TTG AAA TTT GGG GCT CTT GCA ATC TAC TTA GGA GGG CTA GGA ATA GGA ACA TGG  180
41  LYS ILE LEU LYS PHE GLY ALA LEU ALA ILE TYR LEU GLY GLY LEU GLY ILE GLY THR TRP

TCT ACT GGA AGG GTT GCT GCA GGT GGA TCA CCA AGG TAC ACA CCA CTC CGA ACA GCA GGG  240
61  SER THR GLY ARG VAL ALA ALA GLY GLY SER PRO ARG TYR THR PRO LEU ARG THR ALA GLY

TCC ACA TCA TCG CTT GCA TCA ATA GGA TCC AGA GCT GTA ACA GCA GGG ACC CGC CCC AGT  300
81  SER THR SER SER LEU ALA SER ILE GLY SER ARG ALA VAL THR ALA GLY THR ARG PRO SER

ATA GGT GCG GGC ATT CCT TTA GAC ACC CTT GAA ACT CTT GGG GCC TTG CGT CCA GGG GTG  360
101 ILE GLY ALA GLY ILE PRO LEU ASP THR LEU GLU THR LEU GLY ALA LEU ARG PRO GLY VAL

TAT GAG GAC ACT GTG CTA CCA GAG GCC CCT GCA ATA GTC ACT CCT GAT GCT CTT CCT GCA  420
121 TYR GLU ASP THR VAL LEU PRO GLU ALA PRO ALA ILE VAL THR PRO ASP ALA LEU PRO ALA

GAT TCA GGG CTT GAT GCC CTC TCC ATA GGT ACA GAG TCC TCC ACG GAG ACC CTC ATT ACT  480
141 ASP SER GLY LEU ASP ALA LEU SER ILE GLY THR GLU SER SER THR GLU THR LEU ILE THR

CTG CTA GAG CCT GAG GGT CCC GAG GAC ATA GCG GTT CTT GAG CTG CAA CCC CTG GAC CGT  540
161 LEU LEU GLU PRO GLU GLY PRO GLU ASP ILE ALA VAL LEU GLU LEU GLN PRO LEU ASP ARG

                                                              PvuII
CCA ACT TGG CAA GTA AGC AAT GCT GTT CAT CAG TCC TCT GCA TAC CAC GCC CCT CTG CAG  600
181 PRO THR TRP GLN VAL SER ASN ALA VAL HIS GLN SER SER ALA TYR HIS ALA PRO LEU GLN

CTG CAA TCG TCC ATT GCA CAA ACA TCT GGT TTA GAA AAT ATT TTT GTA GGA GGC TCG GGT  660
201 LEU GLN SER SER ILE ALA GLN THR SER GLY LEU GLU ASN ILE PHE VAL GLY GLY SER GLY

TTA GGG GAT ACA GGA GGA GAA AAC ATT GAA CTG ACA TAC TTC GGG TCC CCA CGA ACA AGC  720
221 LEU GLY ASP THR GLY GLY GLU ASN ILE GLU LEU THR TYR PHE GLY SER PRO ARG THR SER

ACG CCC CGC AGT ATT GCC TCT AAA TCA CCT GGC ATT TTA AAC TGG TTC AGT AAA CGG TAC  780
241 THR PRO ARG SER ILE ALA SER LYS SER PRO GLY ILE LEU ASN TRP PHE SER LYS ARG TYR

TAC ACA CAG GTG CCC ACG GAA GAT CCT GAA GTG TTT TCA TCC CAA ACA TTT GCA AAC CCA  840
261 TYR THR GLN VAL PRO THR GLU ASP PRO GLU VAL PHE SER SER GLN THR PHE ALA ASN PRO

     PvuII
CTG TAT GAA GCA GAA CCA GCT GTG CTT AAG GGA CCT AGT GGA CGT GTT GGA CTC AGT CAG  900
281 LEU TYR GLU ALA GLU PRO ALA VAL LEU LYS GLY PRO SER GLY ARG VAL GLY LEU SER GLN

GTT TAT AAA CCT GAT ACA CTT ACA ACA CGT AGC GGG ACA GAG GTG GGA CCA CAG CTA CAT  960
301 VAL TYR LYS PRO ASP THR LEU THR THR ARG SER GLY THR GLU VAL GLY PRO GLN LEU HIS

GTC AGG TAC TCA TTG AGT ACT ATA CAT GAA GAT GTA GAG GCA ATC CCC TAC ACA GTT GAT  1,020
321 VAL ARG TYR SER LEU SER THR ILE HIS GLU ASP VAL GLU ALA ILE PRO TYR THR VAL ASP

# FIG. 3B

```
                                  BglII                              BglII
     AAA GAA ATT AAT GCA AGT AAA TCA GAT CTA CCT CTT GAC ATT CAA AAT GAG ATC TGC TTG  2,100
207  Lys Glu Ile Asn Ala Ser Lys Ser Asp Leu Pro Leu Asp Ile Gln Asn Glu Ile Cys Leu

     TAC CCA GAC TAC CTC AAA ATG GCT GAG GAC GCT GCT GGT AAT AGC ATG TTC TTT TTT GCA  2,160
227  Tyr Pro Asp Tyr Leu Lys Met Ala Glu Asp Ala Ala Gly Asn Ser Met Phe Phe Phe Ala

     AGG AAA GAA CAG GTG TAT GTT AGA CAC ATC TGG ACC AGA GGG GGC TCG GAG AAA GAA GCC  2,220
247  Arg Lys Glu Gln Val Tyr Val Arg His Ile Trp Thr Arg Gly Gly Ser Glu Lys Glu Ala

     CCT ACC ACA GAT TTT TAT TTA AAG AAT AAT AAA GGG GAT GCC ACC CTT AAA ATA CCC AGT  2,280
267  Pro Thr Thr Asp Phe Tyr Leu Lys Asn Asn Lys Gly Asp Ala Thr Leu Lys Ile Pro Ser

                                                                           BglI
     GTG CAT TTT GGT AGT CCC AGT GGC TCA CTA GTC TCA ACT GAT AAT CAA ATT TTT AAT CGC  2,340
287  Val His Phe Gly Ser Pro Ser Gly Ser Leu Val Ser Thr Asp Asn Gln Ile Phe Asn Arg

     CCC TAC TGG CTA TTC CGT GCC CAG GGC ATG AAC AAT GGA ATT GCA TGG AAT AAT TTA TTG  2,400
307  Pro Tyr Trp Leu Phe Arg Ala Gln Gly Met Asn Asn Gly Ile Ala Trp Asn Asn Leu Leu

     TTT TTA ACA GTG GGG GAC AAT ACA CGT GGT ACT AAT CTT ACC ATA AGT GTA CCC TCA GAT  2,460
327  Phe Leu Thr Val Gly Asp Asn Thr Arg Gly Thr Asn Leu Thr Ile Ser Val Pro Ser Asp

     GGA ACC CCA CTA ACA GAG TAT GAT AGC TCA AAA TTC AAT GTA TAC CAT AGA CAT ATG GAA  2,520
347  Gly Thr Pro Leu Thr Glu Tyr Asp Ser Ser Lys Phe Asn Val Tyr His Arg His Met Glu

     GAA TAT AAG CTA GCC TTT ATA TTA GAG CTA TGC TCT GTG GAA ATC ACA GCT CAA ACT GTG  2,580
357  Glu Tyr Lys Leu Ala Phe Ile Leu Glu Leu Cys Ser Val Glu Ile Thr Ala Gln Thr Val

     TCA CAT CTG CAA GGA CTT ATG CCC TCT GTG CTT GAA AAT TGG GAA ATA GGT GTG CAG CCT  2,640
387  Ser His Leu Gln Gly Leu Met Pro Ser Val Leu Glu Asn Trp Glu Ile Gly Val Gln Pro

     CCT ACC TCA TCG ATA TTA GAG GAC ACC TAT CGC TAT ATA GAG TCT CCT GCA ACT AAA TGT  2,700
407  Pro Thr Ser Ser Ile Leu Glu Asp Thr Tyr Arg Tyr Ile Glu Ser Pro Ala Thr Lys Cys

     GCA AGC AAT GTA ATT CCT GCA AAA CAA GAC CCT TAT GCA GGG TTT AAG TTT TGG AAC ATA  2,760
427  Ala Ser Asn Val Ile Pro Ala Lys Gln Asp Pro Tyr Ala Gly Phe Lys Phe Trp Asn Ile

         BglII     pC2VI
                        HindIII
     GAT CTT AAA CAA AAG CTT TCT TTG GAC TTA GAT CAA TTT CCC TTG GGA AGA AGA TTT TTA  2,820
447  Asp Leu Lys Gln Lys Leu Ser Leu Asp Leu Asp Gln Phe Pro Leu Gly Arg Arg Phe Leu

     GCA CAG CAA GGG GCA GGA TGT TCA ACT GTG AGA AAA CGA AGA ATT AGC CAA AAA ACT TCC  2,880
467  Ala Gln Gln Gly Ala Gly Cys Ser Thr Val Arg Lys Arg Arg Ile Ser Gln Lys Thr Ser

     AGT AAG CCT GCA AAA AAA AAA AAA AAA TAA AAG CTA AGT TTC TAT AAA TGT TCT GTA AAT  2,940
487  Ser Lys Pro Ala Lys Lys Lys Lys Lys ***

                  HincII
     GTA AAA CAG AAG GTA AGT CAA CTG CAC CTA ATA AAA ATC ACT TAA TAG                   3,000
                                                                   *** ***
```

# FIG. 3C

GAA AAT ACA CAG GGA CTT GCA TTC GTA CCC TTG CAT GAA GAG CAA GCA GGT TTT GAG GAG 1,080
GLU ASN THR GLN GLY LEU ALA PHE VAL PRO LEU HIS GLU GLU GLN ALA GLY PHE GLU GLU

ATA GAA TTA GAT GAT TTT AGT GAG ACA CAT AGA CTG CTA CCT CAG AAC ACC TCT TCT ACA 1,140
ILE GLU LEU ASP ASP PHE SER GLU THR HIS ARG LEU LEU PRO GLN ASN THR SER SER THR

CCT GTT GGT AGT GGT GTA CGA AGA AGC CTC ATT CCA ACT CAG GAA TTT AGT GCA ACA CGG 1,200
PRO VAL GLY SER GLY VAL ARG ARG SER LEU ILE PRO THR GLN GLU PHE SER ALA THR ARG

CCT ACA GGT GTT GTA ACC TAT GGC TCA CCT GAC ACT TAC TGT GCT AGC CCA GTT ACT GAC 1,260
PRO THR GLY VAL VAL THR TYR GLY SER PRO ASP THR TYR CYS ALA SER PRO VAL THR ASP

pFU41
ClaI
CCT GAT TCT ACC TCT CCT AGT CTA GTT ATC GAT GAG ACT ACT ACT ACA CCA ATC ATT ATA 1,320
PRO ASP SER THR SER PRO SER LEU VAL ILE ASP GLU THR THR THR THR PRO ILE ILE ILE

ATT GAT GGC CAC ACA GTT GAT TTG TAC AGC AGT AAC TAC ACC TTG CAT CCC TCC TTG TTG 1,380
ILE ASP GLY HIS THR VAL ASP LEU TYR SER SER ASN TYR THR LEU HIS PRO SER LEU LEU

LI→
AGG AAA CGA AAA AAA CGG AAA CAT GCC TAA TTT TTT TTG CAG ATG GCG TTG TGG CAA CAA 1,440
ARG LYS ARG LYS LYS ARG LYS HIS ALA ***                MET ALA LEU TRP GLN GLN

GGC CAG AAG CTG TAT CTC CCT CCA ACC CCT GTA AGC AAG GTG CTT TGC AGT GAA ACC TAT 1,500
GLY GLN LYS LEU TYR LEU PRO PRO THR PRO VAL SER LYS VAL LEU CYS SER GLU THR TYR
pBPD18,pC2VI,pBPT307
←
GTG CAA AGA AAA AGC ATT TTT TAT CAT GCA GAA ACG GAG CGC CTG CTA ACT ATA GGA CAT 1,560
VAL GLN ARG LYS SER ILE PHE TYR HIS ALA GLU THR GLU ARG LEU LEU THR ILE GLY HIS

CCA TAT TAC CCA CTG TCT ATC GGG GAC AAA ACT GTT CCT AAG GTC TCT GCA AAT CAG TAT 1,620
PRO TYR TYR PRO LEU SER ILE GLY ASP LYS THR VAL PRO LYS VAL SER ALA ASN GLN TYR

AGG GTA TTT AAA ATA CAA CTA CCT GAT CCC AAT CAA TTT GCA CTA CCT GAC AGG ACT GTT 1,680
ARG VAL PHE LYS ILE GLN LEU PRO ASP PRO ASN GLN PHE ALA LEU PRO ASP ARG THR VAL

CAC AAC CCA AGT AAA GAG CGG CTG GTG TGG GCA GTC ATA GGT GTG CAG GTG TCC AGA GGG 1,740
HIS ASN PRO SER LYS GLU ARG LEU VAL TRP ALA VAL ILE GLY VAL GLN VAL SER ARG GLY

CAG CCT CTT GGA GGT ACT GTA ACT GGG CAC CCC ACT TTT AAT GCT TTG CTT GAT GCA GAA 1,800
GLN PRO LEU GLY GLY THR VAL THR GLY HIS PRO THR PHE ASN ALA LEU LEU ASP ALA GLU

AAT CTG AAT AGA AAA GTC ACC ACC CAA ACA ACA GAT GAC AGG AAA CAA ACA GGC CTA GAT 1,860
ASN LEU ASN ARG LYS VAL THR THR GLN THR THR ASP ASP ARG LYS GLN THR GLY LEU ASP

GCT AAG CAA CAA CAG ATT CTG TTG CTA GGC TGT ACC CCT GCT GAA GGG GAA TAT TGG ACA 1,920
ALA LYS GLN GLN GLN ILE LEU LEU LEU GLY CYS THR PRO ALA GLU GLY GLU TYR TRP THR

ACA GCC CGT CCA TGT GTT ACT GAT CGT CTA GAA AAT GGC GCC TGC CCT CCT CTT GAA TTA 1,980
THR ALA ARG PRO CYS VAL THR ASP ARG LEU GLU ASN GLY ALA CYS PRO PRO LEU GLU LEU

AAA AAC AAG CAC ATA GAA GAT GGG GAT ATG ATG GAA ATT GGG TTT GGT GCA GCC AAC TTC 2,040
LYS ASN LYS HIS ILE GLU ASP GLY ASP MET MET GLU ILE GLY PHE GLY ALA ALA ASN PHE

# FIG. 4

# FIG. 5

# FIG. 6

EUR. COPY

0133123

# FIG. 7

# FIG. 8

# FIG. 9

0133123

FIG. 10

FIG. 11

0133123

13 / 14

FIG. 12

0133123

14/14

European Patent Office

**EUROPEAN SEARCH REPORT**

01.33123

| DOCUMENTS CONSIDERED TO BE RELEVANT | | | EP 84401548.7 |
|---|---|---|---|

| Category | Citation of document with indication, where appropriate, of relevant passages | Relevant to claim | CLASSIFICATION OF THE APPLICATION (Int. Cl.4) |
|---|---|---|---|
| P,X | EP - A1 - 0 092 456 (INSTITUT PASTEUR)<br><br>* Claims 1,8,10 *<br><br>-- | 1,21, 23,24, 51,52, 59,61, 103, 104 | C 12 N 15/00<br>C 12 P 21/00<br>C 07 K 15/00<br>A 61 K 39/12<br>//C 12 R 1:19 |
| D,A | VIROLOGY, vol. 89, 1978, New York, USA<br><br>W.D. LANCASTER et al. "Demonstration of Two Distinct Classes of Bovine Papilloma Virus" pages 372-379<br><br>* Totality *<br><br>-- | 1,2,6, 7,13, 14,20-22,40, 51,59 | |
| D,A | NATURE, vol. 299, no. 5883, October 7, 1982, New York, London<br><br>E.Y.CHEN et al. "The primary structure and genetic organization of the bovine papillomavirus type 1 genome" pages 529-534<br><br>* Totality *<br><br>----- | 1,6,7, 20-22 | TECHNICAL FIELDS SEARCHED (Int. Cl.4)<br><br>C 12 N<br>C 12 P<br>C 07 K<br>A 61 K |

The present search report has been drawn up for all claims

| Place of search | Date of completion of the search | Examiner |
|---|---|---|
| VIENNA | 30-10-1984 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82